# EUROPEAN PATENT APPLICATION

(11) **EP 4 850 612 A1**
(43) Date of publication of application: **09.12.2026**
(21) Application number: 26162531.3
(22) Date of filing: 05.03.2026
(51) Int. Cl.: A61B 10/00, A61B 10/02, A61B 90/00

(54) **SWAB GUARD APPARATUS AND SYSTEM**

(30) Priority: 05.06.2025 US 202519230057; 23.01.2026 US 202619458652
(71) Applicant: AZURE INSTITUTE, San Diego, CA 92121 (US); Acon Laboratories, Inc., San Diego, CA 92121 (US)
(72) Inventor: RING, Christian Michael, San Diego, CA 92121 (US); ZHONG, Linlin, San Diego, CA 92121 (US); LIU, Bingchen, San Diego, CA 92121 (US)
(74) Representative: Forresters IP LLP

(57) **Abstract**

A system includes a swab stick and a guard body. The swab stick has a shaft, a collection head and a receiving body. The guard body has a bore that extends through a longitudinal axis of the guard body and a slit. The collection head is disposed on the shaft at a distal end. The collection head is configured for collecting a sample from a test subject. The receiving body is disposed on the shaft in between the collection head and the proximal end of the shaft. The bore of the guard accepts the shaft and has a shape that geometrically complements a shape of the receiving body to permit the guard body to be removably interlocked with the receiving body. The slit is configured to reduce a force necessary to interlock and de-interlock the guard body and receiving body.

## Description

### CROSS-REFERENCE TO RELATED APPLICATIONS

This application claims priority from U.S. Patent Application No. 19/230,057, entitled "SWAB AND GUARD APPARATUS AND SYSTEM," filed June 5, 2025, and U.S. Patent Application No. 19/458,652, entitled "SWAB GUARD APPARATUS AND SYSTEM" filed on January 23, 2026, which are both incorporated herein by reference in their entirety.

### BACKGROUND

The background description provided herein is for the purpose of generally presenting the context of the disclosure. The work of the presently named inventors, to the extent it is described in this background section, as well as aspects of the description that may not otherwise qualify as prior art at the time of filing, are neither expressly nor impliedly admitted as prior art against the present disclosure.

Retrieving samples from the nasal passages of infants and/or young children may be dangerous due to the softness of nasal tissues such young test subjects. A risk of serious harm to the test subject exists if the sample is taken incorrectly. For example, injury may occur if a sample collection swab may be too deeply inserted into the nasal passage of the test subject. The average nasal cavity may vary as between infants, children and adults. For example, for infants the average nasal cavity may be less than 1.0cm in debt. For other humans, the average nasal cavity may be between about 1.0-1.5cm in depth, but it may be difficult for the average person retrieving a sample to accurately gauge how deep into the cavity the swab has been inserted. Similar issues may present themselves in collecting samples from other passages, cavities or other test locations.

### SUMMARY

The following presents a simplified summary of the present disclosure in order to provide a basic understanding of some aspects of the disclosure. This summary is not an extensive overview of the disclosure. It is not intended to identify key or critical elements of the disclosure or to delineate the scope of the disclosure. The following summary merely presents some concepts of the disclosure in a simplified form as a prelude to the more detailed description provided below.

Aspects are presented in the appended claims.

In a version of the present technology, there is a system having a swab stick with a shaft, a collection head, and a receiving body. The system may also have a guard body with a bore that extends through a longitudinal axis of the guard body. The shaft may have a distal end and a proximal end. The collection head may be disposed on the shaft at the distal end of the shaft, and the collection head may be configured for collecting a sample from a test subject. The receiving body may be disposed on the shaft in between the collection head and the proximal end. The receiving body may include a first protrusion relative to the shaft and a second protrusion relative to the shaft. The first and second protrusions may define a recess. At least one of the first protrusion and the second protrusion may be a cammed body. The bore may be sized and shaped to accept the shaft. The bore may include a first portion, a second portion, and a third portion. The first portion may have a shape that geometrically complements the first protrusion, the second portion may have a shape that geometrically complements the recess, and the third portion may have a shape that geometrically complements the second protrusion. The geometrically complementing portions of the receiving body and the corresponding first protrusion, recess and second protrusion of the receiving body may permit the guard body to be removably interlocked with the receiving body.

In some versions, the test subject may be one of an adult, a child, and an infant, the collection head may be shaped and sized to be inserted into a nasal passage of the test subject, and the collection head may be configured for collecting a sample from said nasal passage.

In some versions, the collection head may include one of a polyurethane sponge, cotton, poly fibers, and flocked fibers.

In some versions, the guard body may include a top end and a bottom end, with a flange disposed at the top end and a skirt disposed at the bottom end.

In some versions, the guard body may include a slot oriented parallel to the longitudinal axis of the guard body. The slot may be sized and shaped to permit the receiving body to be inserted into the bore through the slot.

In some versions, the guard body may include a top end and a bottom end. The guard body may be shaped as a cylinder.

In some versions, the guard body may include a slot oriented parallel to the longitudinal axis of the guard body, the slot may be sized and shaped to permit the receiving body to be inserted into the bore through the slot.

In some versions, the shaft may be one of axial and non-axial.

In some versions, the recess may have the same diameter as the shaft.

In some versions, the receiving body may be formed integrally with the shaft.

In some versions, the first protrusion may be a cammed body and the second protrusion may be a proud collar.

In some versions, the first protrusion may be a cammed body and the second protrusion may be a cammed body.

In some versions, the first protrusion may be inverted with respect to the second protrusion.

In some versions, the first protrusion may have a cammed wall that tapers toward the proximate end of the shaft and the second protrusion may have a cammed wall that tapers toward the distal end of the shaft.

In some versions, the first portion may be a top portion, the second portion may be a waist portion, and the third portion may be a bottom portion. The first portion and the waist portion may define an annular top ledge, and the waist portion and the second portion may define an inverted annular bottom ledge.

In some versions, the top portion may have a diameter, the waist portion may have a diameter, and the bottom portion may have a diameter. The difference between the top portion diameter and the waist portion diameter may form the annular top ledge, and the difference between the waist portion diameter and the bottom portion diameter may form the inverted annular bottom ledge.

In some versions, the first protrusion may be a cammed body having an annular cam ledge, the second protrusion may be a proud collar having an annular collar ledge. When the guard body is interlocked with the receiving body, the proud collar may be disposed in the top portion and the annular collar ledge may be in a position of abutment with the annular top ledge. When the guard body is interlocked with the receiving body, the cammed body may be disposed within the bottom portion with the annular cam ledge proximate the inverted annual bottom ledge.

In some versions, the waist portion may have a waist wall. When the guard body is interlocked with the receiving body, the waist wall may be interlocked with the recess.

In some versions, the first portion may be a top portion, the second portion may be a waist portion, and the third portion may be a bottom portion. The bottom portion may accommodate the cammed body, and one of (a) the bottom portion may not accommodate the other protrusion, and (b) the bottom portion may only partially accommodate the other protrusion.

In some versions, the guard body may have a length such that when the collection head is inserted into the guard body, the length of the guard body may cover one of: (a) the entirety of the collection head, and (b) at least a portion of the collection head.

In some versions of the described technology, the disclosure describes a system having a swab stick with a shaft, a collection head, and a receiving body. The system may also have a guard body with a bore that extends through a longitudinal axis of the guard body and may include a slit. The shaft may have a distal end and a proximal end. The collection head may be disposed on the shaft at the distal end of the shaft, and the collection head may be configured for collecting a sample from a test subject. The receiving body may be disposed on the shaft in between the collection head and the proximal end. The bore may be configured to accept the shaft. The bore may have a shape that geometrically complements a shape of the receiving body to permit the guard body to be removably interlocked with the receiving body. The slit may be configured to reduce a force necessary to interlock and de-interlock the guard body and receiving body.

In some versions, the test subject is one of an adult, a child, and an infant, the collection head is shaped and sized to be inserted into a nasal passage of the test subject and the collection head is configured for collecting a sample from said nasal passage.

In some versions, the guard body includes a top end and a bottom end, and the guard body includes a flange disposed at the top end and a skirt disposed at the bottom end.

In some versions, the slit is disposed in the skirt.

In some versions, the slit is oriented longitudinally with respect to the skirt.

In some versions, the slit extends the length of the skirt.

In some versions, the depth of the slit extends from an exterior-facing surface of the skirt to an interior-facing surface of the skirt.

In some versions, the slit is disposed in the flange.

In some versions, the slit is disposed on an interior-facing surface of the flange.

In some versions, the slit comprises a skirt slit disposed in the skirt and a flange slit disposed in the flange.

In some versions, the skirt slit is oriented longitudinally with respect to the skirt.

In some versions, the skirt slit extends the length of the skirt.

In some versions, the depth of the skirt slit extends from an exterior-facing surface of the skirt to an interior-facing surface of the skirt.

In some versions, the flange slit is disposed on an interior-facing surface of the flange.

In some versions, the skirt slit and the flange slit are aligned and form a common slit cavity.

In some versions, the width of the slit is less than the width of the shaft.

In some versions, the guard body includes a top end and a bottom end, and the guard body is shaped as a cylinder.

In some versions, the slit extends the length of the guard body.

In some versions, the slit is disposed on an interior-facing surface of the guard body.

In some versions, the width of the slit is less than the width of the shaft.

### BRIEF DESCRIPTION OF THE DRAWINGS

The appended claims set forth with particularity certain novel features that are considered characteristic of one or more embodiments of the present disclosure. The embodiments of the disclosure itself; however, both as to its structure and operation together with the additional objects and advantages thereof are best understood through the following description when read, by way of example only, in conjunction with the accompanying drawings. The components in the figures are not necessarily to scale, emphasis instead being placed upon illustrating the principles of the disclosure. In the figures, like reference numerals designate corresponding parts throughout the different views, wherein:
Fig. 1A shows a version of a swab guard system in a detached position in accordance with the disclosure;
Fig. 1B shows the swab guard system of Fig. 1A in an attached position;
Fig. 2A shows a front view of a swab stick of some versions of the swab guard system of Fig. 1A;
Fig. 2B shows a detailed view of a receiving body of some versions of the swab stick as indicated in Fig. 2A;
Fig. 3 shows a top view of a guard of some versions of the swab guard system of Fig. 1A;
Fig. 4A shows a front view of the guard of Fig. 3;
Fig. 4B shows a sectional view of the guard as indicated in Fig. 4A;
Fig. 5A shows a sectional view of the swab guard system of Fig. 1A;
Fig. 5B shows a detailed sectional view of the swab guard system as indicated in Fig. 5A;
Fig. 6A shows the swab guard system of Fig. 1A oriented in an incorrect position;
Fig. 6B shows the swab guard system of Fig. 1A oriented in another incorrect position;
Fig. 6C shows the swab guard system of Fig. 1A oriented in another incorrect position;
Fig. 7A shows another version of a swab guard system in a detached position in accordance with the disclosure;
Fig. 7B shows the swab guard system of Fig. 7A in an attached position;
Fig. 8A shows a perspective view of a guard of some versions of the swab guard system of Fig. 7A;
Fig. 8B shows a top view of the guard of Fig. 8A;
Fig. 8C shows a perspective sectional view of the guard as indicated in Fig. 8B;
Fig. 9A shows a left side view of the guard of Fig. 8A;
Fig. 9B shows a sectional view of the guard as indicated in Fig. 9A;
Fig. 10 shows a detailed sectional view of a portion of the swab guard system of Fig. 7A;
Fig. 11 shows a detailed sectional view of the swab guard system of Fig. 7A oriented in an incorrect position:
Fig. 12 shows another version of a swab guard system in an attached position in accordance with the disclosure;
Fig. 13 shows a side view of a guard of some versions of the swab guard system of Fig. 12 with dashed lines indicating hidden internal features;
Fig. 14A shows a front view of a swab stick of some versions of the swab guard system of Fig. 12;
Fig. 14B shows a detailed view of a receiving body of some versions of the swab stick as indicated in Fig. 14A;
Fig. 15A shows a sectional view of the swab guard system of Fig. 12;
Fig. 15B shows a detailed sectional view of the swab guard system as indicated in Fig. 15A;
Fig. 16A shows another version of a swab guard system in an attached position in accordance with the disclosure;
Fig. 16B shows a perspective view of a guard of some versions of the swab guard system of Fig. 16A;
Fig. 17A shows a front view of the guard of Fig. 16B;
Fig. 17B shows a top view of the guard of Fig. 16B;
Fig. 17C shows a sectional view of the guard as indicated in Fig. 17B;
Fig. 18A shows a sectional view of the swab guard system of Fig. 16A; and
Fig. 18B shows a detailed sectional view of the swab guard system as indicated in Fig. 18A.
Fig. 19A shows a version of a swab guard system in a detached position in accordance with the disclosure.
Fig. 19B shows the swab guard system of Fig. 19A in an attached position.
Fig. 20 shows a front view of the guard of some versions of the swab guard system of Fig. 19A.
Fig. 21 shows a rear view of the guard of Fig. 20. The left side view and right side view of the guard of Fig. 20 is the same as such rear view depicted in Fig. 21.
Fig. 22 shows a top view of the guard of Fig. 20.
Fig. 23 shows a bottom view of the guard of Fig. 20.
Fig. 24 shows a sectional view of the guard as indicated in Fig. 22.
Fig. 25 shows a sectional view of the guard as indicated in Fig. 22.
Fig. 26 shows a sectional view of the guard as indicated in Fig. 22.
Fig. 27A shows aversion of a swab guard system in a detached position in accordance with the disclosure.
Fig. 27B shows the swab guard system of Fig. 27A in an attached position.
Fig. 28 shows a front view of the guard of some versions of the swab guard system of Fig. 27A.
Fig. 29 shows a rear view of the guard of Fig. 28. The left side view and right side view of the guard of Fig. 28 is the same as such rear view depicted in Fig. 29.
Fig. 30 shows a top view of the guard of Fig. 28.
Fig. 31 shows a bottom view of the guard of Fig. 28.
Fig. 32 shows a sectional view of the guard as indicated in Fig. 30.
Fig. 33 shows a sectional view of the guard as indicated in Fig. 30.
Fig. 34 shows a sectional view of the guard as indicated in Fig. 30.

Persons of ordinary skill in the art will appreciate that elements in the figures are illustrated for simplicity and clarity so not all connections and options have been shown to avoid obscuring various aspects of the disclosure. For example, common but well-understood elements that are useful or necessary in a commercially feasible version are not often depicted in order to facilitate a less obstructed view of these various versions of the present disclosure. It will be further appreciated that certain actions and/or steps may be described or depicted in a particular order of occurrence while those skilled in the art will understand that such specificity with respect to sequence is not actually required. It will also be understood that the terms and expressions used herein are to be defined with respect to their corresponding respective areas of inquiry and study except where specific meaning have otherwise been set forth herein.

### DETAILED DESCRIPTION

The present disclosure now will be described more fully hereinafter with reference to the accompanying drawings, which form a part hereof, and which show, by way of illustration, specific exemplary versions by which the disclosure may be practiced. These illustrations and exemplary versions are presented with the understanding that the description and figures are an exemplification of the principles of one or more versions of the disclosure. The disclosure may be embodied in many different forms and should not be construed as limited to the versions set forth herein; rather, these versions are provided so that this disclosure will be thorough and complete, and will fully convey the scope of the disclosure to those skilled in the art. Among other things, the present disclosure may be embodied as methods or devices. The following detailed description is, therefore, not to be taken in a limiting sense.

Throughout the disclosure and claims, the following terms take the meanings explicitly associated herein, unless the context clearly dictates otherwise. The phrase "in one version" as used herein does not necessarily refer to the same version, although it may. Furthermore, the phrase "in another version" as used herein does not necessarily refer to a different version, although it may. Thus, as described below, various versions of the disclosure may be readily combined, without departing from the scope or spirit of the disclosure and/or invention.

In addition, as used herein, the term "or" is an inclusive "or" operator, and is equivalent to the term "and/or," unless the context clearly dictates otherwise. The term "based on" is not exclusive and allows for being based on additional factors not described, unless the context clearly dictates otherwise. In addition, throughout the specification, the meaning of "a," "an," and "the" include plural references. The meaning of "in" includes "in" and "on."

The figures depict versions for purposes of illustration only. One skilled in the art will readily recognize from the following discussion that alternative versions of the structures and methods illustrated herein may be employed without departing from the principles described herein.

The disclosure describes, in some versions, a swab guard system that helps limit or prevent over insertion of the swabs into the nose or nasal cavity or another passage, cavity or other test location of a test subject (e.g., an infant, a child, an adult, etc.). In some versions, the guard system may include a collar or guard removably disposed on a swab stick that may help limit or prevent the swab from being inserted too deeply into the test subject's nose, passage, cavity or other test location of the test subject. In some versions, the guard may be attached to the test swab stick in a "snap-on" manner (e.g., from the side or axially) that may then be removed, such as before adding the swab to an extraction buffer or other post-sampling action. In some versions, the swab guard system may allow for the same swab design to be used for test subjects of all ages, which may reduce or limit the need to create a secondary test kit specifically for infants or other categories of test subjects who may benefit from such having such a guard.

In some versions, the swab guard system may be composed of a swab stick with a sample collection head and a guard removably disposed on the swab stick. In some versions, the sample collection head may be used to collect a sample of secretions from inside a test subject's nostrils, and the swab stick may be configured to receive the guard at a specific position and/or orientation. In some versions, the sample collection head may be made from polyurethane sponge, cotton, poly fibers, flocked fibers, and other materials known to those skilled in the art. The guard may permit the collection of samples while reducing the risk of inserting the collection head too deep into a passage, cavity or other test location (e.g., a nasal cavity) of the test subject, which may otherwise cause injury to the subject. The guard may be particularly useful when the test subject may be relatively small in size or more vulnerable to injury, such as a young child or infant.

The swab guard system may include various configurations of the swab stick and the guard consistent with the teachings of the disclosure. The swab stick may include components or features, such as near the collection head, that may provide for the guard to be attached and reduce the maximum depth that the collection head can be inserted into a passage, cavity or other test location. In some versions, the swab stick may include one or more protrusions and/or recesses (e.g., at a receiving body) that may interlock or otherwise engage with corresponding guard recesses and/or protrusions included on or in the guard. In some versions, the receiving body and guard protrusions and/or recesses may be configured so as to hold the guard in place while also allowing for removal once a sample may be taken from a test subject. Additionally, the swab and guard protrusions and/or recesses may also discourage or prevent incorrect installation of the guard on the swab stick. In some versions, the swab stick may include protrusions and/or recesses that may include multiple installation locations of the guard, such as to provide for use with various ages and/or sizes of test subjects.

As one of skill in the art will recognize from the figures contained herein and accompanying description, various configurations of the swab stick and the guard features may encourage proper installation of the guard on the swab stick, and may provide tactile, audible, or other feedback to a user to confirm that the guard may be installed properly or to indicate that the guard may be installed improperly. For example, in some versions, the guard may only be installed in a single orientation and any attempts to install the guard in an incorrect orientation may result in the guard not locking in place, or in the guard partially or completely covering the collection head. Further, in some versions, the size of the guard may provide for easier holding by a user and may help reduce potential for sample contamination.

In some versions, the guard body includes a slit that may provide the guard body a degree of flexibility so as to reduce a force necessary to interlock and de-interlock the guard body and receiving body.

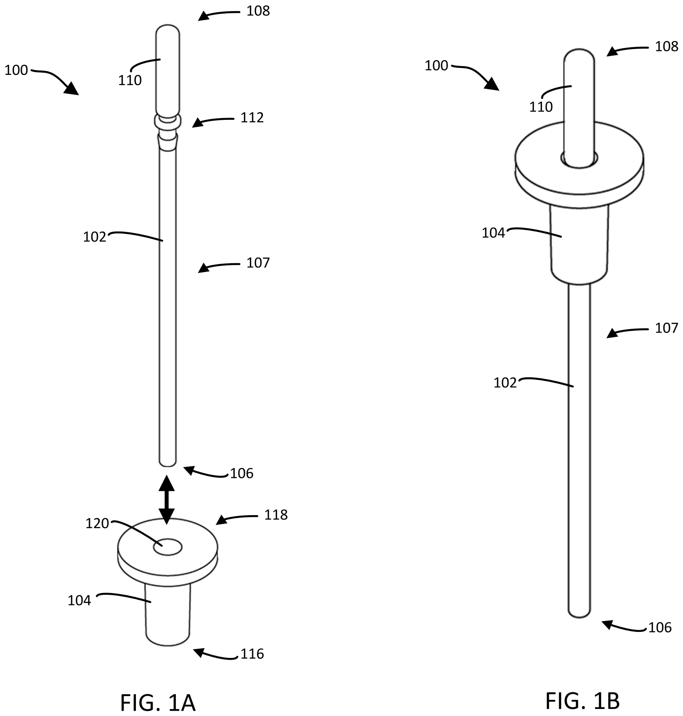

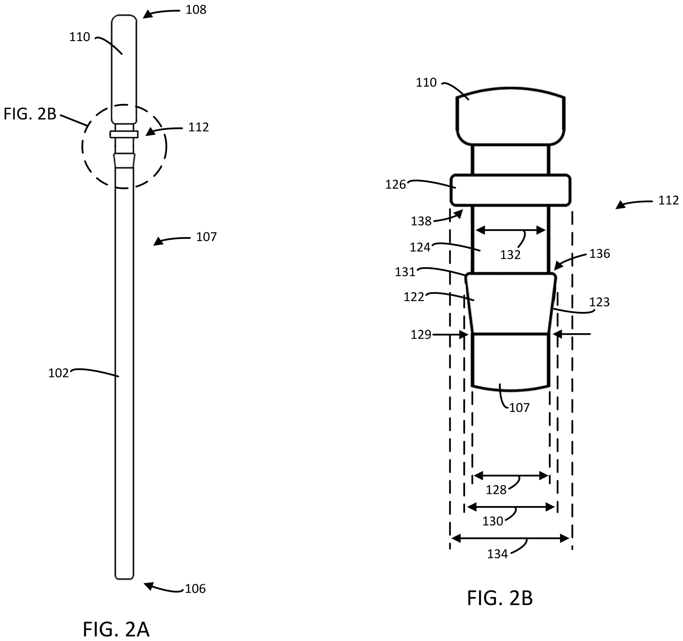

Figs. 1A and 1B show a version of a swab guard system 100 including a swab stick 102 and a nasal guard or guard body 104. Fig. 1A shows an exploded view of the swab guard system 100 with the swab stick 102 and the nasal guard 104 separated and in a detached position, such as prior to or after installation of the nasal guard 104 onto the swab stick. Fig. 1B shows the nasal guard 104 installed onto the swab stick 102 in a proper attached position and orientation to safely and effectively allow for sampling of a test subject. The swab stick 102 may include a base end 106 and a collection end 108 opposite one another and connected by a shaft 107. The collection end 108 may include a collection head 110 that may be configured for insertion into a passage, cavity or other test location of a test subject to take a test sample. The collection head 110 may be made from polyurethane sponge, cotton, poly fibers, flocked fibers, and other materials known to those skilled in the art. The swab stick 102 may also include a receiving body 112 disposed on the shaft 107 between the base end 106 and the collection end 108. In some versions, the receiving body 112 may include one or more recesses, protrusions, and/or other features that may be configured to removably hold the guard 104 in a preferred position with respect to the swab stick 102 and the collection head 110. The features/geometry of the receiving body 112 are described in more detail below with reference to Figs. 2A and 2B. In some versions, the features/geometry of the receiving body 112 may be configured to be or include complimentarily shaped geometry that may interact with corresponding features of the guard 104, which are described in more detail below with reference to Figs. 2-6.

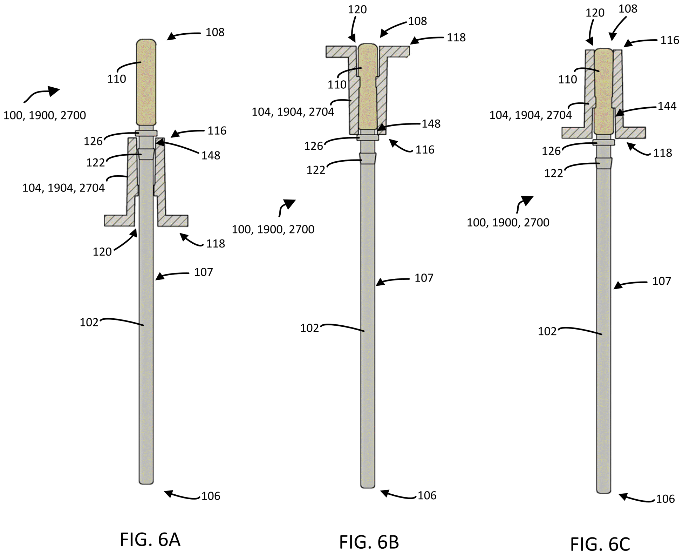

In some versions, the guard or guard body 104 may include a bottom end 116 and a top end 118 opposite one another. A guard bore 120 may be formed longitudinally through the guard 104 between the bottom end 116 and the top end 118. In some versions, the guard bore 120 may be configured to receive the base end 106 of the swab stick 102 therethrough. As shown in Fig. 1B, the guard 104 may be configured to receive the base end 106 of the swab stick 102, slide along the shaft 107, and be disposed around the shaft over the receiving body 112 in an attached position. In the attached position, the top end 118 of the guard 104 may extend toward the collection end 108 of the swab stick 102 so as to expose the entire collection head 110 or to leave exposed a predetermined length of collection head 110. In some versions, the guard 104 and the elements of the receiving body 112 of the swab stick 102 may be configured to interact with one another such that the guard 104 may become secured in place over the receiving body 112 only when the guard 104 is oriented with the top end 118 toward the collection end 108 and the bottom end 116 toward the base end 106. Such positioning may be defined as the "correct orientation" in some versions. In some versions, attempts to orient the guard 104 in an orientation different than the correct orientation (for example, with the top end 118 oriented toward the base end 106 of the swab stick 102) may result in the guard 104 not locking in place on the shaft 107, or the guard 104 covering the entire collection head 110 or a substantial portion of the collection head 110 when in the correct orientation. A guard 104 may cover a substantial portion of the collection head 110 where it does not leave at least a predetermined length of collection head 110 exposed. In other versions, a guard 104 may cover a substantial portion of the collection head 110 where it fails to leave a sufficient length of collection head 110 exposed to collect a sample from a passage, cavity or other test location (e.g., a nasal cavity). In yet another version, a guard 104 may cover a substantial portion of the collection head 110 where it leaves exposed only a nominal length of collection head 110. Additional examples of incorrect orientations of the guard 104 are shown in Figs. 6A, 6B, and 6C.

Fig. 2A shows a side view of the swab stick 102 and Fig. 2B shows an enlarged depiction of the receiving body 112 of the swab stick 102 as indicated in Fig. 2A. In some versions, the receiving body 112 may include a cammed body 122, a recessed body 124, and a proud collar 126 disposed adjacent one another between the base end 106 and the collection head 110. The shaft 107 may have a shaft diameter 128 that may be substantially uniform between the base end 106 and the receiving body 112. The cammed body 122 may have a cam diameter that may vary between a minimum cam diameter 129 where the cammed body 122 meets the shaft 107, and a maximum cam diameter 130 that may be adjacent to the recessed body 124. The minimum cam diameter 129 may be the diameter of the shaft 107. In other versions, the minimum cam diameter 129 may be nominally larger or nominally smaller than the diameter of the shaft 107. Accordingly, in some versions, the cam diameter may uniformly increase (e.g., between the shaft 107 and the recessed body 124) from a minimum diameter 129 and a maximum diameter 130 so as to form a generally frustoconical shape (e.g., between the shaft 107 and the recessed body 124). Although a cam wall 123 of the cammed body 122 shown in Fig. 2B uniformly increases in diameter to provide a substantially conical surface, it is contemplated that, in some versions, the rate of change of the cammed body diameter (e.g., between the shaft 107 and the recessed body 124) may be variable, resulting in a concavely or convexly shaped cam wall surface. The distal most edge 131 of the cammed body 122 may be axially curved to facilitate placement/removal of the guard 104 at the receiving body 112 of swab stick 102.

The recessed body 124 may be formed between the cammed body 122 and the proud collar 126. In some versions, the recessed body 124 may have a substantially uniform recessed diameter 132 between the cammed body 122 and the proud collar 126. In some versions, the recessed diameter 132 may be equal to the shaft diameter 128, but may be different in other versions. In some versions, the recessed diameter 132 may be substantially the same as shaft diameter 128 (e.g., the diameters may deviate only nominally). In some versions, the recessed diameter 132 may be less than the maximum cam diameter 130 such that an annular cam ledge 136 may be formed between (e.g., at the point of abutment of) the cammed body 122 and the recessed body 124. Similarly, the proud collar 126 may have a collar diameter 134 that may be uniform or substantially uniform (e.g., deviating only nominally) and that may be greater than the recessed diameter 132. Accordingly, an inverted annular collar ledge 138 may be formed between (e.g., at the point of abutment of) the recessed body 124 and the proud collar 126. The inverted annular collar ledge 138 may face the annular cam ledge 136. In some versions, the collar diameter 134 may be greater than the maximum cam diameter 130.

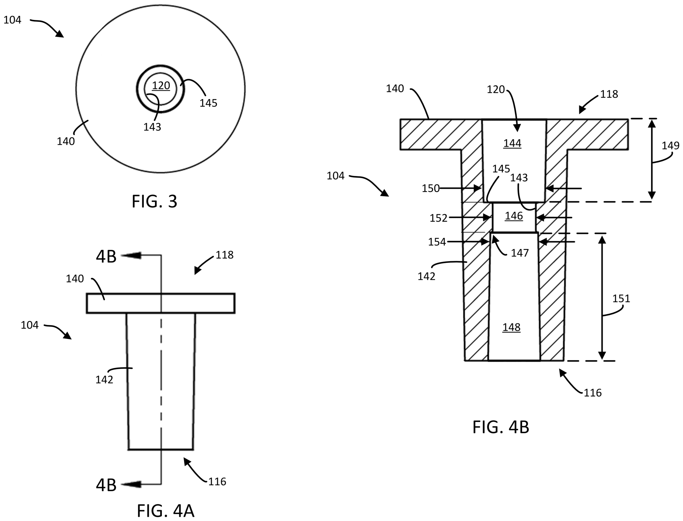

Figs. 3 and 4A show top and side views, respectively, of the guard 104. Fig. 4B shows a sectional view of the guard 104 as indicated in Fig. 4A. The guard 104 may include a flange 140 disposed at the top end 118 and a skirt 142 extending from the flange 140 toward the bottom end 116. The guard bore 120 may be formed through the guard 104 between the top end 118 and the bottom end 116, running through the flange 140 and the skirt 142. As shown in Fig. 4B, the guard bore 120 may include a top portion 144, a waist portion 146, and a bottom portion 148. The top portion 144 may be formed adjacent to the top end 118, the bottom portion 148 may be formed adjacent the bottom end 116, and the waist portion 146 may be formed between the top portion 144 and the bottom portion 148. The top portion 144 may have a top portion diameter 150 that may be greater than a waist portion diameter 152 of the waist portion 146. In some versions, the top portion diameter 150 may be substantially uniform (e.g., differences in diameter are negligible) between the top end 118 and the waist portion 146. In some versions, the top portion diameter 150 may be greater at the top end 118 and may gradually taper towards the waist portion 146. The bottom portion 148 may have a bottom portion diameter 154 that may be greater than the waist portion diameter 152. In some versions, the bottom portion diameter 154 may be substantially uniform (e.g., differences in diameter are negligible) between the waist portion 146 and the bottom end 116. In some versions, the bottom portion diameter 154 may be greater at the bottom end 116 and may gradually taper towards the waist portion 146. In some versions, the difference between the top portion diameter 150 and the waist portion diameter 152 may form an annular top ledge 145 within the guard 104 between (e.g., at the point of abutment of) the top portion 144 and the waist portion 146. Similarly, the difference between the bottom portion diameter 154 and the waist portion diameter 152 may form an inverted annular bottom ledge 147 within the guard 104 between the bottom portion 148 and the waist portion 146. Accordingly, in some versions, the waist portion 146 may include a waist wall or walls 143 (e.g., a cylindrical waist wall) between the top portion 144 and the bottom portion 148 that may define the narrowest diameter of the guard bore 120.

In some versions, the top portion diameter 150 may be sized to accommodate the proud collar 126 within the top portion 144 of the guard bore 120. For example, in some versions, the collar diameter 134 may be less than or equal to the top portion diameter 150 adjacent to the waist portion 146. However, the waist portion diameter 152 may be less than the collar diameter 134 such that the top ledge 145 of the guard 104 may resist or prevent movement of the proud collar 126 into the waist portion 146. Accordingly, in some versions, when the guard 104 is moved from the detached position (Fig. 1A) to the attached position (Fig. 1B), the top ledge 145 of the guard 104 may contact the inverted collar ledge 138 of the receiving body 112 and prevent or resist additional movement of the guard 104 toward the collection end 108 of the swab stick 102. Additionally, in some versions, the collar diameter 134 may be greater than the bottom portion diameter 154 (e.g., where the bottom portion diameter 154 is uniform or substantially uniform) such that the proud collar 126 may not be fully inserted into the lower portion 148 of the guard bore 120. In other versions, the collar diameter 134 may be greater than the maximum bottom portion diameter 154 (e.g., where the bottom portion diameter 154 is tapered) such that the proud collar 126 may not be inserted into the lower portion 148 of the guard bore 120 as is generally depicted in Figs. 6A and 6B. In other versions, the collar diameter 134 may be greater than a bottom portion diameter 154 at a non-zero length from the bottom portion 116 such that the proud collar 126 may not be fully inserted into lower portion 148 of the guard bore 120.

In some versions, the bottom portion diameter 154 may be sized to accommodate the cammed body 122 within the bottom portion 148 of the guard bore 120. For example, in some versions, the maximum cam diameter 130 may be less than or equal to the bottom portion diameter 154 adjacent to the waist portion 146. Further, the waist portion diameter 152 may be greater than or equal to the minimum cam diameter 129 but at least slightly less than the maximum cam diameter 130. Accordingly, in some versions, as the guard 104 moves from the detached position to the attached position (as is suggested by the bidirectional arrow in FIG. 1A), the minimum cam diameter 129 may fit through the waist portion 146 with at least minimal clearance from the waist wall 143. As the guard 104 continues to move toward the collection end 108, the cam wall 123 of the cammed body 122 may contact the waist wall 143 as the diameter of the cammed body 122 increases from the minimum cam diameter 129 to the maximum cam diameter 130. As the diameter of the cammed body 122 increases, the waist wall 143 at the top ledge 145 may begin to interfere with the cam wall 123, resisting further movement of the guard 104 toward the collection end 108. In some versions, when the guard 104 is moved relative to the shaft 107 toward the collection end 108 with enough force to overcome the resistance of the waist wall 143 at the top ledge 145, the cammed body 122 may be pushed past the top ledge 145, through the waist portion 146 and into the bottom portion 148. In other words, in some versions, the maximum diameter 130 of the cammed body 122 may be sized so as to have an interference fit with the waist wall 143 of the waist portion 146 that may be overcome when adequate pressure may be applied. In some versions, as the guard 104 may be forced to overcome the interference fit between the waist wall 143 of the waist portion 146, the guard 104 and/or the waist wall may be configured to flex radially outward away from the shaft 107 so as to accommodate passage of the cammed body 122 through the waist portion 146 and into the bottom portion 148. In such a placement, the annular collar ledge 138 of the proud collar 126 may rest on the annular top ledge 145 of the guard 104 and the inverted annular bottom ledge 147 may rest on or be proximate the annular cam ledge 136 of the cammed body 122.

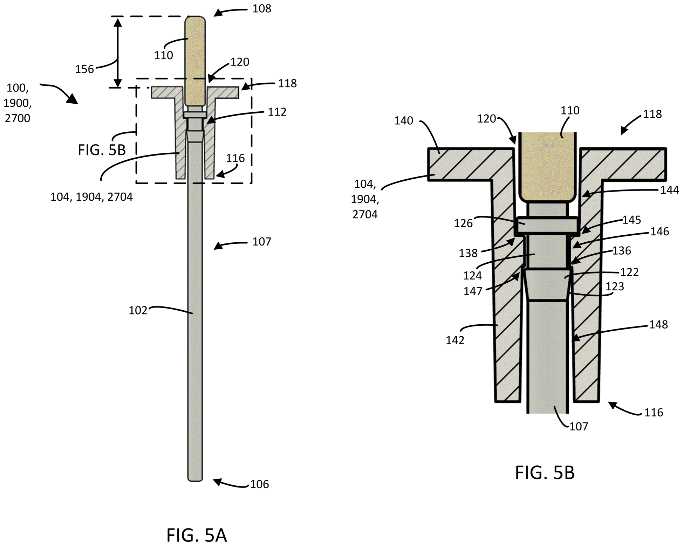

The top portion 144 of the guard bore 120 may have a length 149 that may be measured longitudinally between the top ledge 145 and the top end 118 of the guard 104. Similarly, the bottom portion 148 of the guard bore 120 may have a bottom portion length 151 measured between the inverted bottom ledge 147 and the bottom end 116 of the guard 104. In some versions, as is shown more clearly in Figs. 5A and 5B, the top portion length 149 may determine how much of the collection head 110 may be covered by the guard 104 when installed on the swab stick 102. For example, a longer top portion length 149 may result in more of the collection head 110 being covered by the guard 104 and less of the collection head 110 being exposed above the top end 118 of the guard 104. Conversely, a shorter top portion length 149 may result in less of the collection head 110 being covered by the guard 104 and more of the collection head 110 being exposed above the top end 118 of the guard 104. Accordingly, by varying the top portion length 149, the guard 104 may be used for varying ages and/or sizes of test subjects (e.g., a longer top portion length 149 for smaller test subjects and shorter top portion length 149 for larger test subjects (e.g., subjects older than 6 months old, older than 1 year old, older than 2 years old, etc.)). The significance of the top portion length 149 and the bottom portion length 151 are discussed in more detail with reference to Figs. 6A-C.

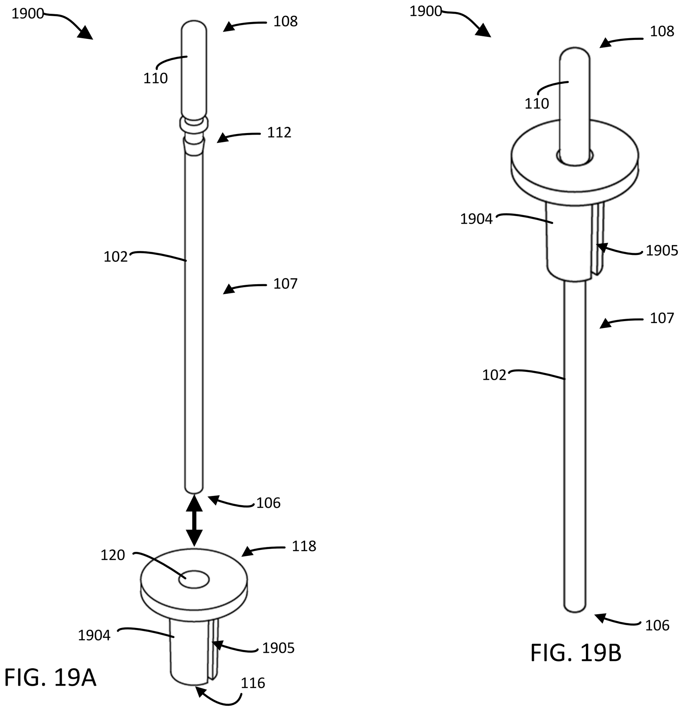

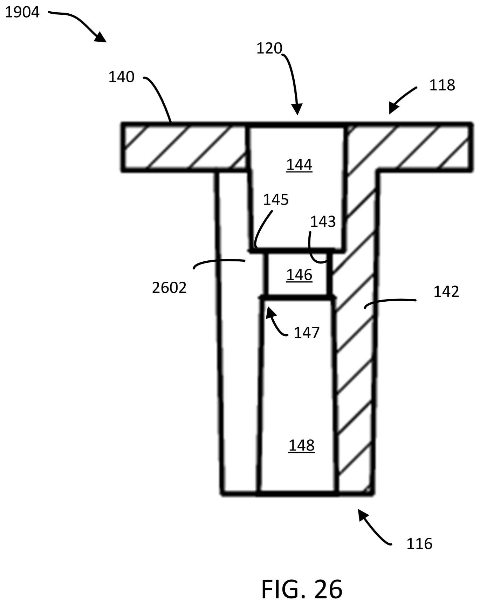

Fig. 5A shows a sectional view of the guard 104, 1904 (discussed below relative to the swab guard system 1900 of Figs. 19A-25), 2704 (discussed below relative to the swab guard system 2700 in Figs. 26A-32) positioned over the receiving body 112 of the swab stick 102 such that the features/geometry of the receiving body may be interlocked with the complimentary interior features/geometry formed within the guard bore 120 of the guard 104, 1904, 2704. Fig. 5B shows a detailed sectional view as indicated in Fig. 5A. As described with reference to Figs 1A and 1B, the guard 104, 1904, 2704 may be configured to slide over the base end 106 of the swab stick 102, slide up the shaft portion 107, and may lock or snap into place at the receiving body 112. In some versions, an interlocking relationship between the external features/geometry of the receiving body 112 and the internal features/geometry of the guard 104, 1904, 2704 may operate to hold the guard 104 in an attached position, such as shown in Fig. 5B.

In some versions, in the attached position, particular features/geometry of the receiving body 112 and the guard 104, 1904, 2704 may be positioned so as to removably hold the guard 104, 1904, 2704 in place. For example, in some versions, in the attached position, the guard 104, 1904, 2704 may be disposed with respect to the shaft 107 such that the recessed body 124 may be disposed within the waist portion 146 of the guard bore 120. In the attached position, the inverted collar ledge 138 of the proud collar 126 may abut or be adjacent to the top ledge 145 of the guard 104, 1904, 2704 such that the inverted collar ledge 138 may prohibit or limit further movement of the guard 104, 1904, 2704 toward the collection head 110. Conversely, the cam ledge 136 of the cammed body 122 may abut or be adjacent to the inverted bottom ledge 147 of the guard 104, 1904, 2704 such that the cam ledge 136 may resist or limit movement of the guard 104, 1904, 2704 toward the base end 106 of the swab stick 102.

As described with reference to Fig. 4B, in some versions, the guard 104, 1904, 2704 may be installed onto the swab stick 102 by inserting the base end 106 of the swab stick into the guard bore 120. The guard 104, 1904, 2704 may then be moved relative to the shaft 107 from the base end 106 toward the collection end 108. Those skilled in the art will recognize that, alternatively or additionally, moving the swab stick 102 relative to the guard 104, 1904, 2704 may have the same result as moving the guard 104, 1904, 2704 relative to the swab stick. In some versions, as the guard 104, 1904, 2704 moves toward the collection end 108, the cammed body 122 of the receiving body 112 may pass through the top portion 144 of the guard bore 120 and the waist wall 143 at the top ledge 145 may encounter the cam wall 123 of the cammed body 122. As the diameter of the cammed body 122 increases from the minimum cam diameter 129 to the maximum cam diameter 130, additional movement of the guard 104, 1904, 2704 may encounter growing resistance. In some versions, when adequate force may be used to move the guard 104, 1904, 2704 toward the collection end 108, the resistance may be overcome and the cammed body 122 may be received into the lower portion 148 of the guard bore 120, as shown in Fig. 5B. In some versions, when the guard 104, 1904, 2704 may be forced to overcome the interference fit and be disposed in the attached position, an audible or tactile indicator may be heard or felt, respectively, by the user to indicate that the guard 104, 1904, 2704 may have been properly installed.

In some versions, removing the guard 104, 1904, 2704 from the attached position to the detached position may be achieved by applying adequate force to the guard 104, 1904, 2704 toward the base end 108 of the swab stick 102 to overcome an interference fit between the cam ledge 136 of the cammed body 122 and the inverted bottom ledge 147 of the guard 104, 1904, 2704. In some versions, the guard 104, 1904, 2704 and/or the waist wall 143 may be configured to flex radially outward away from the shaft 107 so as to accommodate passage of the cammed body 122 through the waist portion 146 and into the bottom portion 148 (for attachment) or passage into the top portion 144 (for detachment).

In the attached position, shown in Figs. 5A and 5B, at least a portion of the collection head 110 may extend clear of the top end 118 of the guard 104, 1904, 2704, leaving that portion exposed and ready for collecting a specimen from a test subject. An exposed portion 156 may be the amount of the collection head 110 that may be inserted into a passage, cavity or other test location (e.g., nasal cavity) of a test subject before being prohibited or resisted from further insertion by the flange 140. Accordingly, the guard 104, 1904, 2704 may act to prevent insertion of the collection head 110 too far into a passage, cavity or other test location (e.g., nasal cavity) of a test subject. In some versions, the overall length of the guard 104, 1904, 2704 or at least the top portion length 149 of the guard 104, 1904, 2704 may be sized based on how much of the collection head 110 may constitute/should constitute the exposed portion 156. For example, in some versions, the exposed portion 156 may be between 50 percent and 90 percent of the collection head 110, or may be between 55 percent and 85 percent in other versions, or between 60 and 80 percent in other versions. In some versions, the exposed portion 156 may be between 30 percent and 70 percent of the collection head 110, or between 40 and 60 percent in other versions.

Figs. 6A, 6B, and 6C demonstrate how some versions of the swab guard system 100, 1900, 2700 may provide one or more indicators that the guard 104, 1904, 2704 may have been installed incorrectly. For example, Fig. 6A shows the guard 104, 1904, 2704 installed in an upside-down position, with the bottom end 116 more proximate the collection end 108 than the top end 118. In some versions, when the base end 106 of the swab stick 102 is inserted into the guard bore 120 through the bottom end 116 of the guard 104, 1904, 2704 (instead of through the top end 118), the guard 104, 1904, 2704 may be moved toward the collection end 108 with respect to the shaft 107 such that the cammed body 122 may be received at least partially within the lower portion 148 of the guard bore 120. However, because the proud collar 126 may have a proud collar diameter 134 that may be greater than a bottom portion diameter 154, the proud collar 126 may prevent or resist further movement of the guard 104, 1904, 2704 toward the collection end 108. Depending on the materials used in manufacture, the cammed body 122 may further prevent/resist movement of the guard 104, 1904, 2704 toward the collection end 108. Accordingly, when installed upside-down, the guard 104, 1904, 2704 may not cover any portion of the collection head 110, which should indicate to a user that the guard 104, 1904, 2704 may be installed improperly. Additionally, due to the bottom portion length 151, the collars and ledges within the guard 104, 1904, 2704 and on the swab stick 102 may not interact with one another. Accordingly, the guard 104, 1904, 2704 may not be held in place and may fall off of the swab stick 102 altogether.

Fig. 6B illustrates a scenario where the guard 104, 1940, 2704 in the correct orientation may have been installed over the collection end 108 of the swab stick 102 instead of over the base end 106. In such an occurrence, the guard bore 120 may receive the collection head 110 within the guard 104, 1904, 2704. However, because the proud collar 126 may have a proud collar diameter 134 that may be greater than a bottom portion diameter 154, the proud collar may prevent or resist further movement of the guard 104, 1904, 2704 toward the base end 106. Additionally, in some versions, a diameter of the collection head 110 may be greater than the guard bore 120 diameter and may therefore resist movement of the guard 104, 1904, 2704 toward the base end 106. In the scenario of Fig. 6B, a user may be alerted to the incorrect positioning of the guard 104, 1904, 2704 on the swab stick 102 because either no portion of the collection head 110 or a relatively small portion of the collection head 110 may be exposed clear of the guard 104, 1904, 2704, which may prevent effective sampling of the test subject. A user may further be alerted to the incorrect positioning of the guard 104 due to the degree of difficulty in sliding the guard 104, 1904, 2704 over the collection head 110.

Fig. 6C illustrates a scenario where the guard 104, 1904, 2704 may have been installed over the collection end 108 of the swab stick 102 instead of over the base end 106 and where the guard 104, 1904, 2704 may have been installed upside-down. In such a scenario, a diameter of the collection head 110 may be greater than the guard bore 120 diameter and may therefore resist movement of the guard 104, 1904, 2704 toward the base end 106. Additionally, even if the proud collar 126 may be received within the top portion 144 of the guard bore 120, the guard 104, 1904, 2704 may be prevented or resisted from moving further toward the base end 106 when the top ledge 145 of the guard 104, 1904, 2704 contacts the proud collar 126. In the scenario of Fig. 6C, a user may be alerted to the incorrect positioning of the guard 104, 1904, 2704 on the swab stick 102 because either no portion of the collection head 110 or a relatively small portion of the collection head 110 may be exposed clear of the guard 104, 1904, 2704, which may prevent effective sampling of the test subject. A user may further be alerted to the incorrect positioning of the guard 104, 1904, 2704 due to the degree of difficulty in sliding the guard 104, 1904, 2704 over the collection head 110.

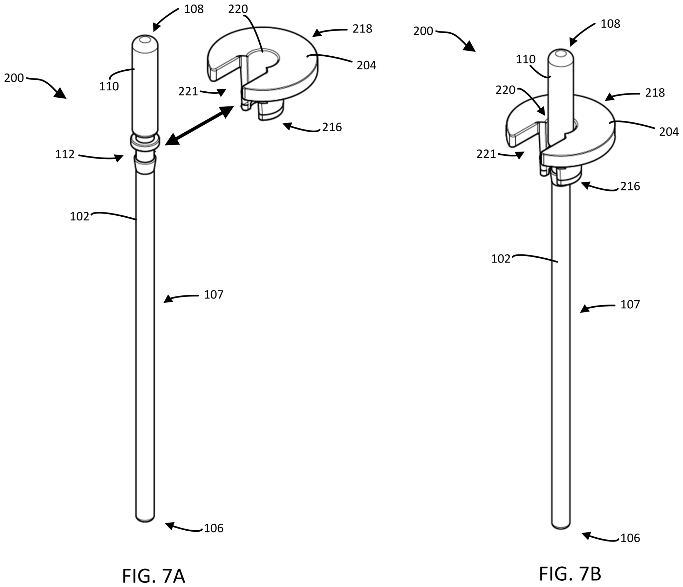

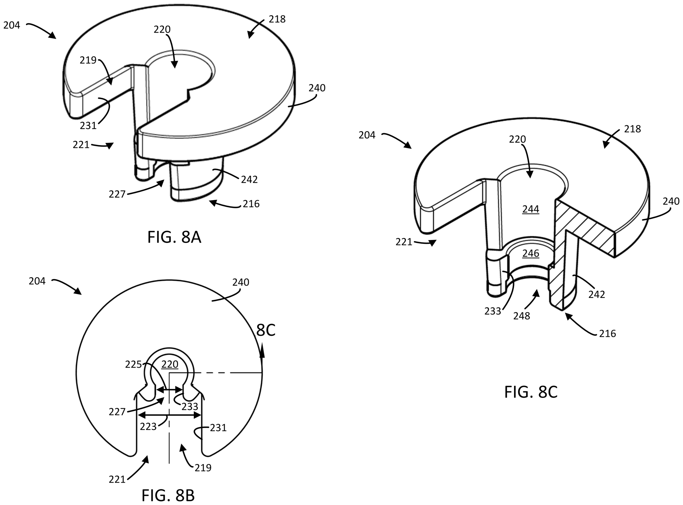

Figs. 7A and 7B show another version of a swab guard system 200 including the swab stick 102 and a guard 204. In some versions, the swab stick 102 may be substantially similar or identical to the swab stick 102 described with reference to Figs. 1-6, with like reference numbers representing like features. Fig. 7A shows the swab guard system 200 in a detached position with the guard 204 and the swab stick 102 separate from one another. Fig. 7B shows the swab guard system 200 in an attached position, with the guard 204 disposed over and around the receiving body 112 of the swab stick 102. In some versions, the features/geometry of the receiving body 112 may be configured to be complimentarily shaped recesses, collars, and cams that may interact with corresponding features of the guard 204, which are described in more detail below with reference to Figs. 8-9.

In some versions, the guard or guard body 204 may include a bottom end 216 and a top end 218 opposite one another. A guard bore 220 may be formed longitudinally through the guard 204 between the bottom end 216 and the top end 218. A guard slot 221 may be formed into the guard body204, providing lateral access to the guard bore 220 from the bottom end 216 to the top end 218 of the guard body204. In some versions, the guard slot 221 may be configured to receive the swab stick 102 so as to laterally insert the swab stick into the guard bore 220. In some versions, the guard bore 220 may also be configured to receive the base end 106 of the swab stick 102 therethrough, similar to guard 104 described with reference to Figs. 1-6.

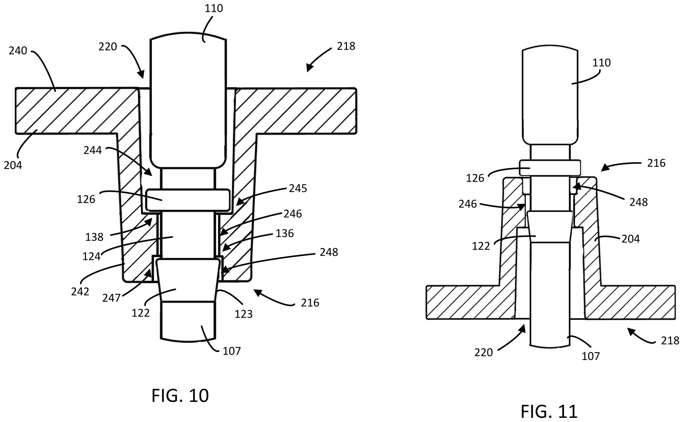

In the attached position, such as shown in Fig. 7B, the top end 218 of the guard 204 may extend toward the collection end 108 of the swab stick 102 so as to at least partially cover the collection head 110. In some versions, the guard 204 and the elements of the receiving body 112 of the swab stick 102 may be configured to interact with one another such that the guard 204 may become secured in place over and around the receiving body 112 only when the guard 204 is oriented with the top end 218 toward the collection end 108 and the bottom end 216 toward the base end 106. Such positioning may be defined as the "correct orientation" in some versions. In some versions, attempts to orient the guard 204 in an orientation different than the correct orientation (for example, with the top end 218 oriented toward the base end 106 of the swab stick 102) may result in the guard 204 not locking in place on the shaft 107, or the guard 204covering the entire collection head 110 or a substantially larger portion of the collection head than when in the correct orientation. An example of incorrect orientations of the guard 204 is shown in Fig. 11.

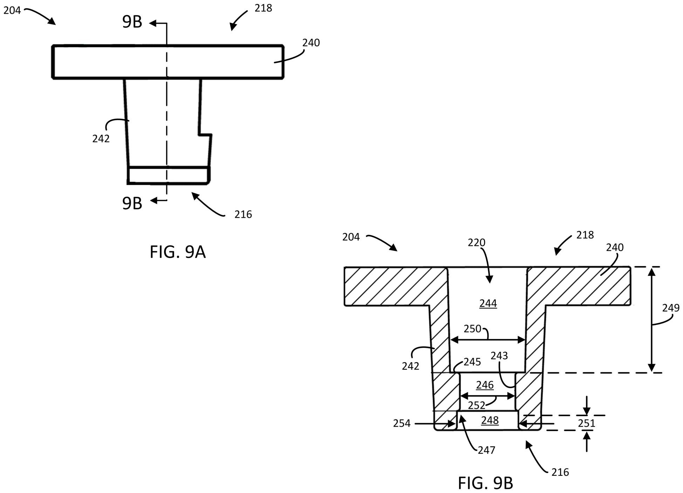

Figs. 8A-C and 9A-B show various views of the guard 204. Figs. 8A and 8B show perspective and top views, respectively, of the guard 204. Fig. 8C shows a sectional view of the guard 204 as indicated in Fig. 8B. The guard 204 may include a flange 240 disposed at the top end 218 and a skirt 242 extending from the flange toward the bottom end 216. The guard bore 220 may be formed through the guard 204 between the top end 218 and the bottom end 216, running through the flange 240 and the skirt 242. As shown in Fig. 8C, the guard bore 220 may include a top portion 244, a waist portion 246, and a bottom portion 248. The top portion 244 may be formed adjacent to the top end 218, the bottom portion 248 may be formed adjacent the bottom end 216, and the waist portion 246 may be formed between the top portion and the bottom portion.

In some versions, the guard slot 221 may include a flange slot 219 formed into the flange 240 and a skirt slot 227 formed into the skirt 242. The flange slot 219 may provide lateral access to the guard bore 220 through the flange 240, and the skirt slot 227 may provide lateral access to the guard bore through the skirt 242. The flange slot 219 may be defined by substantially parallel flange slot walls 231, which may define a flange slot width 223. Similarly, the skirt slot 227 may be defined by substantially parallel skirt slot walls 233, which may define a skirt slot width 225. In some versions, the skirt slot walls 233 may be disposed at or adjacent the waist portion 246 of the guard bore 220. In some versions, the flange slot width 223 may be greater than the shaft diameter 128 and/or a diameter of the collection head 110 such that the shaft 107 and/or the collection head may be received within the flange slot 219. In some versions, the skirt slot width 225 may be similar or slightly less than to the shaft diameter 128 so that the shaft 107 may be received into the skirt slot 227 with an interference fit. In some versions, skirt slot walls 233 may flex outwardly away from one another to accommodate the shaft 107 being forced radially inward toward the guard bore 220. In some versions, when the guard 204 may be forced to overcome the interference fit and be disposed in the attached position, an audible or tactile indicator may be heard or felt, respectively, by the user to indicate that the guard 204 may have been properly installed.

Fig. 9A shows a side view of the guard 204, and Fig. 9B shows a sectional side view of the guard 204 as indicated in Fig. 9A. The top portion 244 may have a top portion diameter 250 that may be greater than a waist portion diameter 252 of the waist portion 246. In some versions, the top portion diameter 250 may be substantially uniform between the top end 218 and the waist portion 246. In some versions, the top portion diameter 250 may be greater at the top end 218 and may gradually taper towards the waist portion 246. The bottom portion 248 may have a bottom portion diameter 254 that may be greater than the waist portion diameter 252. In some versions, the difference between the top portion diameter 250 and the waist portion diameter 252 may form an annular top ledge 245 within the guard 204 between the top portion 244 and the waist portion 246. Similarly, the difference between the bottom portion diameter 254 and the waist portion diameter 252 may form an annular inverted bottom ledge 247 within the guard 204 between the bottom portion 248 and the waist portion 246. Accordingly, in some versions, the waist portion 246 may include a waist wall or walls 243 (e.g., a cylindrical waist wall) between the top portion 244 and the bottom portion 248 that may define the narrowest diameter of the guard bore 220.

In some versions, the top portion diameter 250 may be sized to accommodate the proud collar 126 of the swab stick 102 within the top portion 244 of the guard bore 220. For example, in some versions, the collar diameter 134 of the proud collar 126 may be less than or equal to the top portion diameter 250 adjacent to the waist portion 246. However, the waist portion diameter 252 may be less than the collar diameter 134 such that the top ledge 245 of the guard 204 may resist or prevent movement of the proud collar 126 into the waist portion 246. Accordingly, in some versions, when the guard 104 is moved from the detached position (Fig. 7A) to the attached position (Fig. 7B), the top ledge 245 of the guard 204 may contact the inverted collar ledge 138 of the receiving body 112 and prevent or resist movement of the guard toward the collection end 108 of the swab stick 102. Additionally, in some versions, the collar diameter 134 may be greater than the bottom portion diameter 254 such that the proud collar 126 may not be inserted into the lower portion 248 of the guard bore 220.

In some versions, the bottom portion diameter 254 may be sized to accommodate the cammed body 122 of the swab stick 102 within the bottom portion 248 of the guard bore 220. For example, in some versions, the maximum cam diameter 130 may be less than or equal to the bottom portion diameter 254 adjacent to the waist portion 246. Further, the waist portion diameter 252 may be greater than or equal to the minimum cam diameter 129 but at least slightly less than the maximum cam diameter 130. Accordingly, in some versions, as the guard 204 may be moved upward towards the collection end 108 from a position between the base end 106 and the receiving body 112, the minimum cam diameter 129 may fit through the waist portion 246 with at least minimal clearance from the waist wall 243. As the guard 204 continues to move toward the collection end 108, the cam wall 123 of the cammed body 122 may contact the waist wall 243 as the diameter of the cammed body 122 increases from the minimum cam diameter 129 to the maximum cam diameter 130. As the diameter of the cammed body 122 increases, the geometry of the top ledge 245 may begin to interfere with the cam wall 123, resisting further movement of the guard 204 toward the collection end 108. In some versions, when the guard 204 is moved relative to the shaft 107 toward the collection end 108 with enough force to overcome the resistance of the top ledge 245, the cammed body 122 may be pushed past the waist portion 246 and into the bottom portion 248. In other words, in some versions, the maximum diameter 130 of the cammed body 122 may be sized so as to have an interference fit with the waist wall 243 of the waist portion 246 that may be overcome when adequate pressure may be applied. In some versions, as the guard 204 may be forced to overcome the interference fit between the waist wall 243 of the waist portion 246, the guard 204 and/or the waist wall 243 may be configured to flex radially outward away from the shaft 107 so as to accommodate passage of the cammed body 122 through the waist portion 246 and into the bottom portion 248.

The top portion 244 of the guard bore 220 may have a top portion length 249 that may be measured longitudinally between the top ledge 245 and the top end 228 of the guard 204. Similarly, the bottom portion 248 of the guard bore 220 may have a bottom portion length 251 measured between the inverted bottom ledge 247 and the bottom end 216 of the guard 204. In some versions, as is shown more clearly in Fig. 10, the top portion length 249 may determine how much of the collection head 110 may be covered by the guard 204 when installed on the swab stick 102. For example, a longer top portion length 249 may result in more of the collection head 110 being covered by the guard 204 and less of the collection head 110 being exposed above the top end 218 of the guard. Conversely, a shorter top portion length 249 may result in less of the collection head 110 being covered by the guard 204 and more of the collection head 110 being exposed above the top end 218 of the guard 204. Accordingly, by varying the top portion length 249, the guard 204 may be used for varying ages and/or sizes of test subjects (i.e., longer top portion length for smaller test subjects and shorter top portion length for larger test subjects).

Fig. 10 shows a partial sectional view of the guard 204 positioned over the receiving body 112 of the swab stick 102 such that the features/geometry of the receiving body may be interlocked with the complementary interior features/geometry formed within the guard bore 220 of the guard 204. As described with reference to Figs. 7A and 7B, the guard 204 may be configured to be laterally "snapped" onto the swab stick 102 by fitting the swab stick into the guard slot 221. In some versions, the guard 204 may be laterally attached to the swab stick 102 in position over the receiving body 112 as shown in Fig. 10. In some versions, the guard 204 may be snapped into position virtually anywhere on the shaft 107 and then be moved toward the collection end 108 until it may be snapped in place over the receiving body similar to the guard 104 shown in Figs. 1-6. In some versions, the guard 204 may slide over the base end 106 of the swab stick 102, slide up the shaft portion 107, and may lock or snap into place at the receiving body 112. In some versions, an interlocking relationship between the external features of the receiving body 112 and the internal features of the guard 204 may operate to hold the guard 104 in an attached position, such as shown in Fig. 10.

In some versions, in the attached position, particular features of the receiving body 112 and the guard 204 may be positioned so as to removably hold the guard 204 in place. For example, in some versions, in the attached position, the guard 204 may be disposed with respect to the shaft 107 such that the recessed body 124 may be disposed within the waist portion 246 of the guard bore 220. In the attached position, the inverted collar ledge 138 of the proud collar 126 may abut or be adjacent to the top ledge 245 of the guard 204 such that the inverted collar ledge 138 may prohibit or limit further movement of the guard 204 toward the collection head 110. Conversely, the cam ledge 136 of the cammed body 122 may abut or be adjacent to the inverted bottom ledge 247 of the guard 204 such that the cam ledge 136 may resist or limit movement of the guard 204 toward the base end 106 of the swab stick 102.

In some versions, the guard 204 may be installed onto the swab stick 102 by inserting the base end 106 of the swab stick into the guard bore 220. The guard 204 may then be moved relative to the shaft 107 from the base end 106 toward the collection end 108. Those skilled in the art will recognize that, alternatively or additionally, moving the swab stick 102 relative to the guard 204 may have the same result as moving the guard 204 relative to the swab stick. In some versions, as the guard 204 moves toward the collection end 108, the cammed body 122 of the receiving body 112 may pass through the top portion of the guard bore 220 and the top ledge 245 may encounter the cam wall 123 of the cammed body. As the diameter of the cammed body 122 increases from the minimum cam diameter 129 to the maximum cam diameter 130, additional movement of the guard 204 may encounter growing resistance. In some versions, when adequate force may be used to move the guard 204 toward the collection end 108, the resistance may be overcome and the cammed body 122 may be received into the lower portion 248 of the guard bore 220, as shown in Fig. 10. In some versions, as the resistance from the cammed body 112 is overcome, the guard 204 may flex radially outward to accommodate passage of the cammed body into the lower portion 248 of the guard bore 220.

In some versions, removing the guard 204 from the attached position to the detached position may be achieved by applying adequate lateral force to the guard 204 laterally away from the swab stick 102 to overcome an interference fit between the recessed body 124 and the skirt slot walls 233. In some versions, the guard 204 and/or the skirt slot walls 233 may be configured to flex radially outward away from the shaft 107 so as to accommodate passage of the recessed body 124 through the skirt slot walls 233 to detach from the swab stick 102. In some versions, removing the guard 204 may be alternatively or additionally achieved by applying adequate force to the guard 204 toward the base end 108 of the swab stick 102 to overcome an interference fit between the cam ledge 136 of the cammed body 122 and the inverted bottom ledge 247 of the guard 204. In some versions, the guard 204 and/or the waist wall may be configured to flex radially outward away from the shaft 107 so as to accommodate passage of the cammed body 122 through the waist portion 246 and into the bottom portion 248 (for attachment) or passage into the top portion 244 (for removal).

In the attached position, shown in Fig. 10, at least a portion of the collection head 110 may extend clear of the top end 218 of the guard 204, leaving that portion exposed and ready for collecting a specimen from a test subject, which may be defined as an exposed portion of the collection head 110. The exposed portion may be the amount of the collection head 110 that may be inserted into a passage, cavity or other test location (e.g., nasal cavity) of a test subject before being prohibited or resisted from further insertion by the flange 240. Accordingly, the guard 204 may act to protect from inserting the collection head 110 too far into a passage, cavity or other test location (e.g., nasal cavity) of a test subject. In some versions, the overall length of the guard 204 or at least the top portion length 249 of the guard 204 may be sized based on how much of the collection head 110 may be left as the exposed portion. For example, in some versions, the exposed portion may be between 50 percent and 90 percent of the collection head 110, or may be between 55 percent and 85 percent in other versions, or between 60 and 80 percent in other versions. In some versions, the exposed portion 156 may be between 30 percent and 70 percent of the collection head 110, or between 40 and 60 percent in other versions.

Fig. 11 demonstrates how some versions of the swab guard system 200 may provide one or more indicators that the guard 204 may have been installed incorrectly. Generally, in some versions, the waist portion 246 of the guard 204 may be sized so as to only fit onto the swab stick 102 in a single direction (i.e., right side up). In some versions, the bottom portion length 251 may be too small to allow the guard 204 to be properly seated when incorrectly oriented. For example, the guard 204 in Fig. 11 is installed in an upside-down position, with the bottom end 216 between the collection head 110 and the top end 218. In some versions, when the base end 106 of the swab stick 102 is inserted into the guard bore 220 through the bottom end 216 of the guard 204 (instead of the top end 218), the guard 204 may be moved toward the collection head 110 with respect to the shaft 107 such that the cammed body 122 may be received at least partially within the lower portion 248 and/or the waist portion 246 of the guard bore. However, because the proud collar 126 may have a proud collar diameter 134 that may be greater than the bottom portion diameter 254, the proud collar may prevent or resist further movement of the guard 204 toward the collection head 110. Accordingly, when installed upside-down, the guard 204 may not cover any portion of the collection head 210, which should indicate to a user that the guard 204 may be installed improperly. Additionally, due to the bottom portion length 251, the collars and ledges within the guard 204 and on the swab stick 102 may not interact with one another. Accordingly, the guard 204 may not be held in place and may fall off of the swab stick 102 altogether. Similarly, if the upside-down guard 204 may be laterally moved from a detached position to an attached position, the complimentary features within the guard 204 may not hold the guard 204 in place on the swab stick 102.

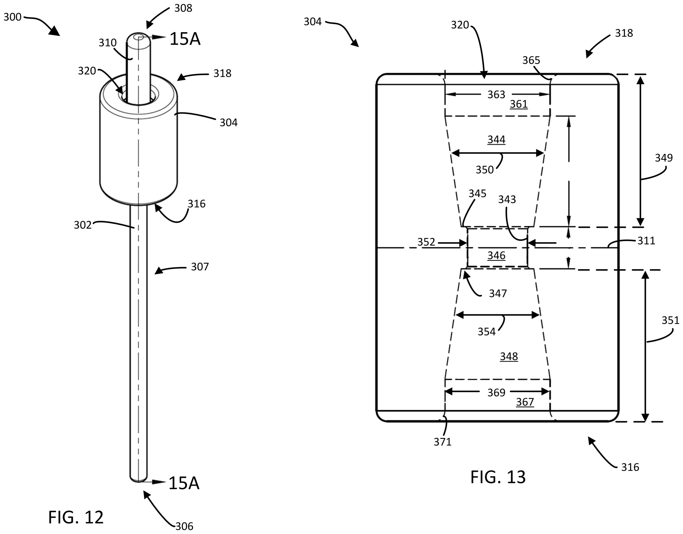

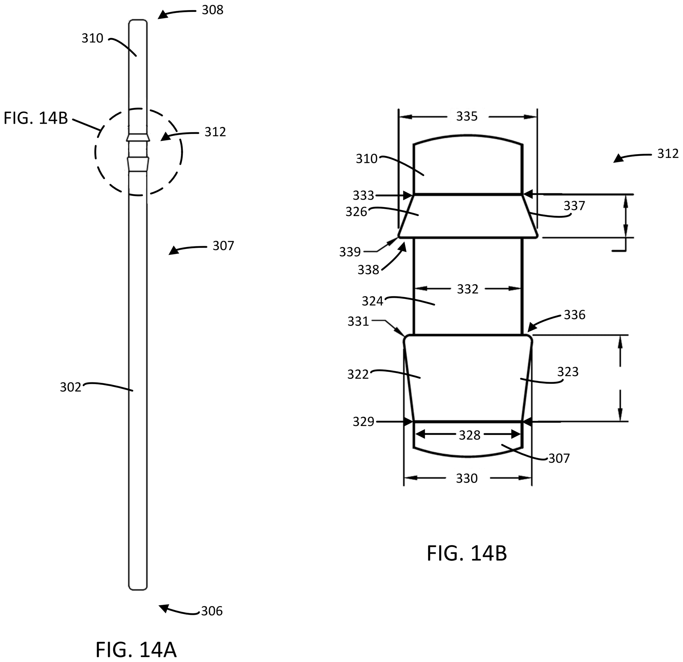

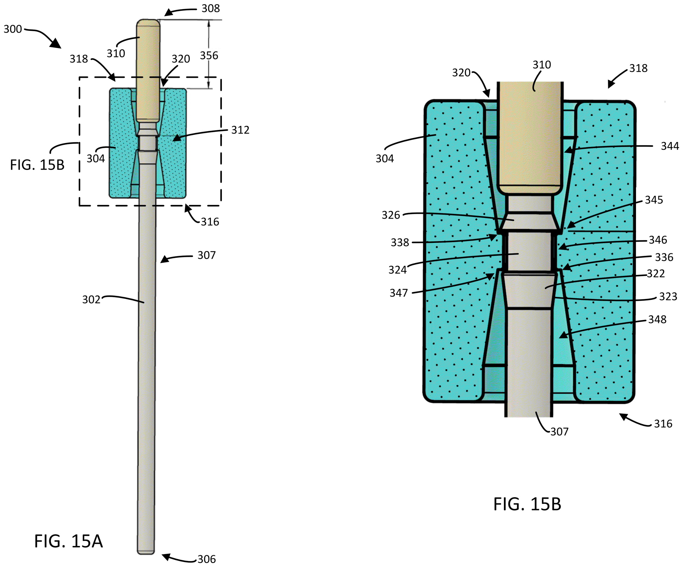

Fig. 12 shows another version of a swab guard system 300 including the swab stick 302 and a guard 304. Fig. 12 shows the guard 304 in an attached position, installed onto the swab stick 102. The guard 304 may be slidable along the swab stick 302 into a detached position separate from the swab stick 302. The swab stick 302 may include a base end 306 and a collection end 308 opposite one another and connected by a shaft 307. The collection end 308 may include a collection head 310 that may be configured for insertion into a passage, cavity or other test location (e.g., nasal cavity) of a test subject to take a test sample. The swab stick 302 may also include a receiving body 312 (Fig. 14) disposed on the shaft 307 between the base end 306 and the collection end 308. In some versions, the receiving body 312 may include one or more recesses, protrusions, cams, and/or other features that may be configured to removably hold the guard 304 in a preferred position with respect to the swab stick 302 and the collection head 302. The features/geometry of the receiving body 312 are described in more detail below with reference to Figs. 14A and 14B. In some versions, the features/geometry of the receiving body 312 may be configured to be complimentarily shaped recesses, collars, and cams that may interact with corresponding features of the guard 304, which are described in more detail below with reference to Figs. 13, 15A, and 15B.

In some versions, the guard or guard body 304 may be cylindrical with a first end 318 and a second end 316 opposite one another. A guard bore 320 may be formed longitudinally through the guard 304 between the second end 316 and the first end 318. In some versions, the guard bore 320 may be configured to receive the base end 306 of the swab stick 302 therethrough. The guard 304 may be configured to receive the base end 306 of the swab stick 302, slide along the shaft 307, and be disposed around the shaft over the receiving body 312 in an attached position. In the attached position, the first end 318 of the guard 304 may extend toward the collection end 308 of the swab stick 302 so as to at least partially cover the collection head 310. In some versions, the guard 304 may be symmetrical about a horizontal central axis 311. In such symmetrical versions, user errors in installing the guard 304 to the swab stick 302 may be reduced because either the second end 316 or the first end 318 may be oriented in the "up" position (i.e., more proximate the collection end 308). Accordingly, if a user inserts the base end 308 of the swab stick 302 into the guard bore 320 at either the second end 316 or the first end 318, the guard 304 may be positioned properly for safely collecting a sample from a test subject.

Fig. 14A shows a side view of the swab stick 302 and Fig. 14B shows an enlarged depiction of the receiving body 312 of the swab stick 302 as indicated in Fig. 14A. In some versions, the receiving body 312 may include a bottom cammed body 322, a recessed body 324, and a top cammed body 326 disposed adjacent one another between the base end 306 and the collection head 310. The shaft 307 may have a shaft diameter 328 that may be uniform between the base end 306 and the receiving body 312. The bottom cammed body 322 may have a bottom cam diameter that may vary between a minimum bottom cam diameter 329 where the cammed body meets the shaft, and a maximum bottom cam diameter 330 that may be adjacent to the recessed body 324. Accordingly, in some versions, the bottom cam diameter may uniformly increase (e.g., between the shaft 307 and the recessed body 324) between a minimum bottom cam diameter 329 and a maximum bottom cam diameter 330 so as to form a generally frustoconical shape (e.g., between the shaft and the recessed body). Although a bottom cam wall 323 of the bottom cammed body 322 shown in Fig. 14B uniformly increases in diameter to provide a substantially conical surface, it is contemplated that, in some versions, the rate of change of the bottom cammed body diameter (e.g., between the shaft 307 and the recessed body 324) may be variable, resulting in a concavely or convexly shaped bottom cam wall surface. In some versions, the distal most bottom edge 331 of the bottom cammed body 322 may be axially curved to facilitate placement of the guard 304 at the receiving body 312 of swab stick 302.

The recessed body 324 may be formed between the bottom cammed body 322 and the top cammed body 326. In some versions, the recessed body 324 may have a substantially uniform recessed diameter 332 between the bottom cammed body 322 and the top cammed body 326. In some versions, the recessed diameter 332 may be equal to the shaft diameter 328, but may be different in other versions. In some versions, the recessed diameter 332 may be substantially the same as shaft diameter 328 (e.g., the diameters may deviate only nominally). In some versions, the recessed diameter 332 may be less than the maximum bottom cam diameter 330 such that an annular bottom cam ledge 336 may be formed between (e.g., at the point of abutment of) the bottom cammed body 322 and the recessed body 324.

In some versions, the top cammed body 326 may have a top cammed body diameter that may vary between a minimum bottom cam diameter 333 where the top cammed body 326 meets the collection head 310, and a maximum top cam diameter 335 that may be adjacent to the recessed body 324. Accordingly, in some versions, the top cam diameter may uniformly increase between the collection head 310 and the recessed body 324 between a minimum top cam diameter 333 and a maximum top cam diameter 335 so as to form a generally frustoconical shape between the collection head 310 and the recessed body. In some versions, the frustoconical shape of the top cammed body 326 may be inverted in orientation with respect to the bottom cammed body 322. Although a top cam wall 337 of the top cammed body 322 shown in Fig. 14B uniformly increases in diameter between the collection head 310 and the recessed body 324 to provide a substantially conical surface, it is contemplated that, in some versions, the rate of change of the top cammed body diameter between the collection head 310 and the recessed body 324 may be variable, resulting in a concavely or convexly shaped top cam wall surface.

In some versions, the maximum top cammed body diameter 335 of the top cammed body 326 may have may be greater than the recessed diameter 332. Accordingly, an inverted annular cam ledge 338 may be formed between (e.g., at the point of abutment of) the recessed body 324 and the top cammed body 326. The inverted annular cam ledge 338 may face the annular bottom cam ledge 336. In some versions, the maximum top cammed body diameter 335 may be greater than the maximum bottom cam diameter 330. In some versions, the distal most top edge 339 of the top cammed body 322 may be axially curved to facilitate placement of the guard 304 at the receiving body 312 of swab stick 302. In some versions, a radius of curvature of the distal most bottom edge 331 of the bottom cammed body 326 may be greater than a radius of curvature of the distal most top edge 339 of the top cammed body.

Referring to Fig. 13, the guard bore 320 may be formed through the guard 304 between the second end 316 and the first end 318. As shown in Fig. 4B, the guard bore 320 may include a first portion 344, a waist portion 346, and a second portion 348. The first portion 344 may be formed adjacent to the first end 318, the second portion 348 may be formed adjacent the second end 316, and the waist portion 346 may be formed between the first portion 344 and the second portion 348. The first portion 344 may have a first portion diameter 350 that may be greater than a waist portion diameter 352 of the waist portion 346. In some versions, the first portion diameter 350 may be substantially uniform (e.g., differences in diameter are negligible) between the first end 318 and the waist portion 346. In some versions, the first portion diameter 350 may be greater at the first end 318 and may gradually taper towards the waist portion 346. In some versions, the first portion 344 of the guard bore 320 may include a first entry portion 361 that may be immediately adjacent the first end 318 of the guard 304. In some versions, the first entry portion 361 may have a first entry portion diameter 363 that may be equal to a maximum diameter of the first portion 344. In some versions, the guard 304 may include an annular first fillet 365 between the first end 318 of the guard 304 and the first entry portion 361.

The second portion 348 may have a second portion diameter 354 that may be greater than the waist portion diameter 352. In some versions, the second portion diameter 354 may be substantially uniform (e.g., differences in diameter are negligible) between the waist portion 346 and the second end 316. In some versions, the second portion diameter 354 may be greater at the second end 316 and may gradually taper towards the waist portion 346. In some versions, the second portion 348 of the guard bore 320 may include a second entry portion 367 that may be immediately adjacent the second end 316 of the guard 304. In some versions, the second entry portion 367 may have a second entry portion diameter 369 that may be equal to a maximum diameter of the second portion 348. In some versions, the guard 304 may include an annular second fillet 371 between the second end 316 of the guard 304 and the second entry portion 367.

In some versions, the difference between the first portion diameter 350 and the waist portion diameter 352 may form an annular top ledge 345 within the guard 304 between (e.g., at the point of abutment of) the first portion 344 and the waist portion 346. Similarly, the difference between the second portion diameter 354 and the waist portion diameter 352 may form an inverted annular bottom ledge 347 within the guard 304 between the second portion 348 and the waist portion 346. Accordingly, in some versions, the waist portion 346 may include a waist wall or waist walls 343 (e.g., a cylindrical waist wall) between the first portion 344 and the second portion 348 that may define the narrowest diameter of the guard bore 320.

In some versions, the first portion diameter 350 may be sized to accommodate the top cammed body 326 within the first portion 344 of the guard bore 320. For example, in some versions, the maximum top cam diameter 335 may be less than or equal to the first portion diameter 350 adjacent to the waist portion 346. However, the waist portion diameter 352 may be less than the maximum top cam diameter 335 such that the top ledge 345 of the guard 304 may resist or prevent movement of the top cammed body 326 into the waist portion 346. Accordingly, in some versions, when the guard 304 is moved from the detached position to the attached position (Fig. 12), the top ledge 345 of the guard 304 may contact the inverted annular cam ledge 338 of the receiving body 312 and prevent or resist additional movement of the guard 304 toward the collection end 308 of the swab stick 302.

In some versions, the second portion diameter 354 may be sized to accommodate the bottom cammed body 322 within the second portion 348 of the guard bore 320. For example, in some versions, the maximum bottom cam diameter 330 may be less than or equal to the second portion diameter 354 adjacent to the waist portion 346. Further, the waist portion diameter 352 may be greater than or equal to the minimum cam diameter 329 but at least slightly less than the maximum bottom cam diameter 330. Accordingly, in some versions, as the guard 304 moves from the detached position to the attached position (from off of the swab stick 302 into the attached position depicted in Fig. 12), the minimum bottom cam diameter 329 may fit through the waist portion 346 with at least minimal clearance from the waist wall 343. As the guard 304 continues to move toward the collection end 308, the cam wall 323 of the bottom cammed body 322 may contact the waist wall 343 as the diameter of the bottom cammed body 322 increases from the minimum bottom cam diameter 329 to the maximum bottom cam diameter 330. As the diameter of the cammed body 322 increases, the waist wall 343 at the top ledge 345 may begin to interfere with the bottom cam wall 323, resisting further movement of the guard 304 toward the collection end 308. In some versions, when the guard 304 is moved relative to the shaft 307 toward the collection end 308 with enough force to overcome the resistance of the waist wall 343 at the top ledge 345, the bottom cammed body 322 may be pushed past the top ledge 345, through the waist portion 346 and into the bottom portion 348. In other words, in some versions, the maximum bottom cam diameter 330 of the cammed body 322 may be sized so as to have an interference fit with the waist wall 343 of the waist portion 346 that may be overcome when adequate pressure may be applied. In some versions, as the guard 304 may be forced to overcome the interference fit between the waist wall 343 of the waist portion 346, the guard 304 and/or the waist wall 343 may be configured to flex radially outward away from the shaft 307 so as to accommodate passage of the bottom cammed body 322 through the waist portion 346 and into the second portion 348. In such a placement, the inverted annular cam ledge 338 of the top cammed body 126 may rest on the annular top ledge 345 of the guard 304 and the inverted annular bottom ledge 347 may rest on or be proximate the annular bottom cam ledge 336 of the bottom cammed body 322.

The first portion 344 of the guard bore 320 may have a first length 349 that may be measured longitudinally between the top ledge 345 and the first end 338 of the guard 304. Similarly, the second portion 348 of the guard bore 320 may have a second length 351 measured between the inverted bottom ledge 347 and the second end 316 of the guard 304. In some versions, as is shown more clearly in Figs. 15A and 15B, the first length 349 may determine how much of the collection head 310 may be covered by the guard 304 when installed on the swab stick 302. For example, a longer first length 349 may result in more of the collection head 310 being covered by the guard 304 and less of the collection head 310 being exposed above the first end 318 of the guard 304. Conversely, a shorter first length 349 may result in less of the collection head 310 being covered by the guard 304 and more of the collection head 310 being exposed above the first end 318 of the guard 304. Accordingly, by varying the first length 349, the guard 304 may be used for varying ages and/or sizes of test subjects (e.g., a longer first length 349 for smaller test subjects and shorter top portion length 349 for larger test subjects).

Fig. 15A shows a sectional view of the guard 304 positioned over the receiving body 312 of the swab stick 302 such that the features/geometry of the receiving body 312 may be interlocked with the complimentary interior features formed within the guard bore 320 of the guard 304. Fig. 15B shows a detailed sectional view as indicated in Fig. 15A. In some versions, the guard 304 may be configured to slide over the base end 306 of the swab stick 302, slide up the shaft portion 307, and may lock or snap into place at the receiving body 312. In some versions, an interlocking relationship between the external features of the receiving body 312 and the internal features of the guard 304 may operate to hold the guard 304 in an attached position, such as shown in Fig. 15B.

In some versions, in the attached position, particular features of the receiving body 312 and the guard 304 may be positioned so as to removably hold the guard 304 in place. For example, in some versions, in the attached position, the guard 304 may be disposed with respect to the shaft 307 such that the recessed body 324 may be disposed within the waist portion 346 of the guard bore 320. In the attached position, the inverted annular cam ledge 338 of the top cammed portion 326 may abut or be adjacent to the top ledge 345 of the guard 304 such that the inverted annular cam ledge 338 may prohibit or limit further movement of the guard 304 toward the collection head 310. Conversely, the bottom cam ledge 336 of the bottom cammed body 322 may abut or be adjacent to the inverted annular bottom ledge 347of the guard 304 such that the bottom cam ledge 336 may resist or limit movement of the guard 304 toward the base end 306 of the swab stick 302.

As described with reference to Fig. 13, in some versions, the guard 304 may be installed onto the swab stick 302 by inserting the base end 306 of the swab stick into the guard bore 320. The guard 304 may then be moved relative to the shaft 307 from the base end 306 toward the collection end 308. Those skilled in the art will recognize that, alternatively or additionally, moving the swab stick 302 relative to the guard 304 may have the same result as moving the guard 304 relative to the swab stick 302. In some versions, as the guard 304 moves toward the collection end 308, the bottom cammed body 322 of the receiving body 312 may pass through the first portion 344 of the guard bore 320 and the waist wall 343 at the top ledge 345 may encounter the bottom cam wall 323 of the bottom cammed body 322. As the diameter of the bottom cammed body 322 increases from the minimum bottom cam diameter 329 to the maximum bottom cam diameter 330, additional movement of the guard 304 may encounter growing resistance. In some versions, when adequate force may be used to move the guard 304 toward the collection end 308, the resistance may be overcome and the bottom cammed body 322 may be received into the second portion 348 of the guard bore 320, as shown in Fig. 15B. In some versions, when the guard 304 may be forced to overcome the interference fit and be disposed in the attached position, an audible or tactile indicator may be heard or felt, respectively, by the user to indicate that the guard 304 may have been properly installed.

In some versions, removing the guard 304 from the attached position to the detached position may be achieved by applying adequate force to the guard 304 toward the base end 308 of the swab stick 302 to overcome an interference fit between the bottom cam ledge 336 of the bottom cammed body 322 and the inverted bottom ledge 347 of the guard 304. In some versions, the guard 304 and/or the waist wall 343 may be configured to flex radially outward away from the shaft 307 so as to accommodate passage of the bottom cammed body 322 through the waist portion 346 and into the bottom portion 348 (for attachment) or passage into the top portion 344 (for detachment).

In the attached position, shown in Figs. 15A and 15B, at least a portion of the collection head 310 may extend clear of the first end 318 of the guard 304, leaving that portion exposed and ready for collecting a specimen from a test subject. An exposed portion 356 may be the amount of the collection head 310 that may be inserted into a passage, cavity or other test location (e.g., nasal cavity) of a test subject before being prohibited or resisted from further insertion by the first end 318 of the guard 304. Accordingly, the guard 304 may act to prevent insertion of the collection head 310 too far into a passage, cavity or other test location (e.g., nasal cavity) of a test subject. In some versions, the overall length of the guard 304 or at least the first length 349 of the guard 304 may be sized based on how much of the collection head 310 may constitutes/should constitute the exposed portion 356. For example, in some versions, the exposed portion 356 may be between 50 percent and 90 percent of the collection head 310, or may be between 55 percent and 85 percent in other versions, or between 60 and 80 percent in other versions. In some versions, the exposed portion 356 may be between 30 percent and 70 percent of the collection head 310, or between 40 and 60 percent in other versions.

In some versions, because the guard 304 (including the guard bore 320) may be symmetrical about the horizontal axis 311, those skilled in the art will recognize that the descriptions of the relationships or interactions between components and features on the first end 318 of the guard 304 may be equivalent to corresponding relationships and interactions with the components and features on the second end 316. For example, those skill in the art will recognize that if the guard 304 is installed onto the swab stick 302 by inserting the base end 306 into the second portion 348 of the guard bore 320 (as opposed to the first portion 344 as described above), the interaction of the components in the second portion 348 of the guard bore 320 would be the same or similar to as described above with reference to the first portion 344.

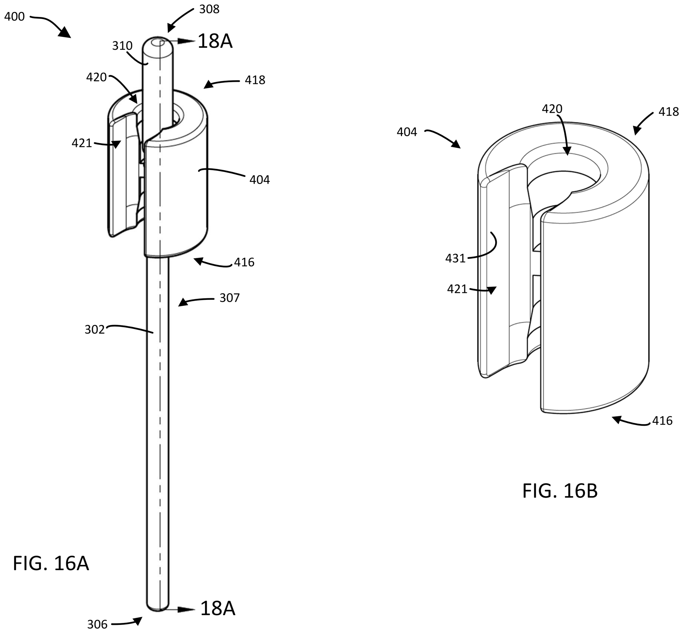

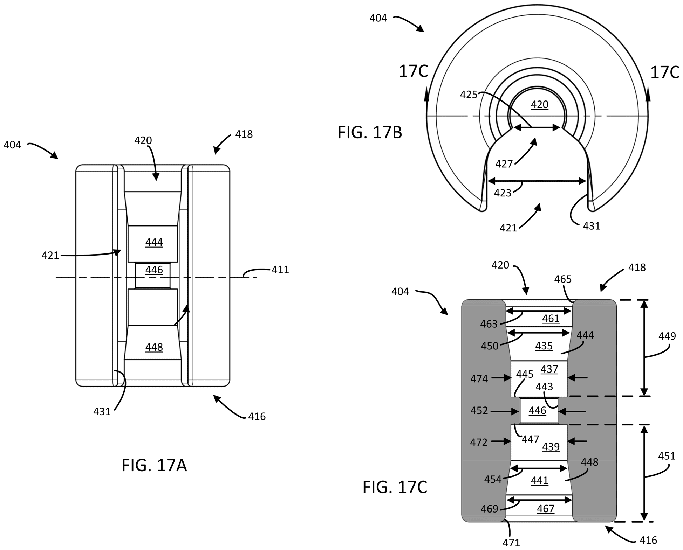

Figs. 16A shows another version of a swab guard system 400 including the swab stick 402 and a guard 404, and Fig. 16B shows perspective view of the guard 404. In some versions, the swab stick 402 may be substantially similar or identical to the swab stick 302 described with reference to Figs. 14A and 14B, with like reference numbers representing like features. Fig. 16A shows the swab guard system 400 in an attached position, with the guard 404 disposed over and around the receiving body 312 of the swab stick 302. In some versions, the guard 404 may be slidable along the swab stick 302 into a detached position separate from the swab stick 302, or may be removed in a lateral direction from the attached position to the detached position. In some versions, the features/geometry of the receiving body 312 may be configured to be complimentarily shaped recesses, collars, and cams that may interact with corresponding features of the guard 404, which are described in more detail below with reference to Figs. 17-18.

In some versions, the guard or guard body 404 may include a first end 418 and a second end 416 and opposite one another. A guard bore 420 may be formed longitudinally through the guard 404 between the first end 418 and the second end 418. A guard slot 421 may be formed into the guard body 404, providing lateral and/or radial access to the guard bore 420 from the first end 418 to the second end 416 of the guard body 404. In some versions, the guard slot 421 may be configured to receive the swab stick 302 so as to laterally insert the swab stick into the guard bore 420. In some versions, the guard bore 420 may also be configured to receive the base end 306 of the swab stick 302 therethrough, similar to guard 304 described with reference to Figs. 12-15.

In the attached position, such as shown in Fig. 16A, the first end 418 of the guard 404 may extend toward the collection end 308 of the swab stick 302 so as to at least partially cover the collection head 310. In some versions, the guard 404 may be symmetrical about a horizontal central axis 411. In such symmetrical versions, user errors in installing the guard 404 to the swab stick 302 may be reduced because either the second end 416 or the first end 418 may be oriented in the "up" position (i.e., more proximate the collection end 308). Accordingly, if a user inserts the base end 308 of the swab stick 302 into the guard bore 420 at either the second end 416 or the first end 418, the guard 404 may be positioned properly for safely collecting a sample from a test subject.

Figs. 17A-C show various views of the guard 404. Fig. 17A shows a front elevation view and 17B shows top view of the guard 404. Fig. 17C shows a sectional view of the guard 404 as indicated in Fig. 17B. The guard bore 420 may be formed through the guard 404 between the first end 418 and the second end 416. The guard bore 420 may include a first portion 444, a waist portion 446, and a second portion 448. The first portion 444 may be formed adjacent to the first end 418, the second portion 448 may be formed adjacent the second end 416, and the waist portion 446 may be formed between the first portion 444 and the second portion 448.

In some versions, the guard slot 421 may provide lateral access to the guard bore 420 through the guard body 404. The guard slot 421 may be defined by substantially parallel slot walls 431, which may define a guard slot width 423. In some versions, the waist portion 446 of the guard bore 420 may represent the most narrow access point into the guard bore 420 through the guard slot 421 at a waist slot 427. In some versions, the waist slot 427 may have a waist slot width 425. In some versions, the slot width 431 may be greater than the shaft diameter 328 and/or a diameter of the collection head 310 such that the shaft 307 and/or the collection head 310 may be received within the slot 421. In some versions, the waist slot width 425 may be similar or slightly less than the shaft diameter 328 so that the shaft 307 may be received into the waist slot 427 with an interference fit. In some versions, the guard 404 may flex away from the shaft 307 to accommodate the shaft 307 being forced radially inward toward the guard bore 420. In some versions, when the guard 404 may be forced to overcome the interference fit and be disposed in the attached position, an audible or tactile indicator may be heard or felt, respectively, by the user to indicate that the guard 404 may have been properly installed.

The first portion 444 of the guard bore 420 may have a first portion diameter 450 that may be greater than a waist portion diameter 452 of the waist portion 446. In some versions, the first portion diameter 450 may be substantially uniform (e.g., differences in diameter are negligible) between the first end 418 and the waist portion 446. In some versions, the first portion diameter 450 may be greater at the first end 418 and may gradually taper towards the waist portion 446. The second portion 448 may have a second portion diameter 454 that may be greater than the waist portion diameter 452. The second portion 448 may have a second portion diameter 454 that may be greater than the waist portion diameter 452. In some versions, the second portion diameter 454 may be substantially uniform (e.g., differences in diameter are negligible) between the waist portion 446 and the second end 416. In some versions, the second portion diameter 454 may be greater at the second end 416 and may gradually taper towards the waist portion 446.

In some versions, the difference between the first portion diameter 450 and the waist portion diameter 452 may form an annular top ledge 445 within the guard 404 between the top portion 444 and the waist portion 446. Similarly, the difference between the second portion diameter 454 and the waist portion diameter 452 may form an annular inverted bottom ledge 447 within the guard 404 between the bottom portion 448 and the waist portion 446. Accordingly, in some versions, the waist portion 446 may include a waist wall or walls 443 (e.g., a cylindrical waist wall) between the first portion 444 and the second portion 448 that may define the narrowest diameter of the guard bore 420.

In some versions, the first portion 444 of the guard bore 420 may include a top section 461, a middle section 435, and a lower section 437. The top section 461 may be disposed adjacent the first end 418 and may have a substantially uniform top section diameter 463 (e.g., differences in diameter are negligible). The lower section 437 may be disposed between the waist section 446 and the middle section 435 and may have a substantially uniform lower section diameter 474 (e.g., differences in diameter are negligible). The middle section 435 may be greater diameter proximate the first end 418 (adjacent the top section 461) and may gradually taper towards the lower portion 437. Similarly, the second portion 448 of the guard bore 420 may include a top section 439, a middle section 441, and a lower section 467. The lower section 467 may be disposed adjacent the second end 416 and may have a substantially uniform lower section diameter 469 (e.g., differences in diameter are negligible). The top section 439 may be disposed between the waist section 446 and the middle section 441 and may have a substantially uniform top section diameter 472 (e.g., differences in diameter are negligible). The middle section 441 may be greater diameter proximate the second end 418 (adjacent the lower section 467) and may gradually taper towards the top portion 439.

In some versions, the first portion diameter 450 may be sized to accommodate the top cammed body 326 within the first portion 444 of the guard bore 420. For example, in some versions, the maximum top cam diameter 335 may be less than or equal to the first portion diameter 450 adjacent to the waist portion 446. However, the waist portion diameter 452 may be less than the maximum top cam diameter 335 such that the top ledge 445 of the guard 404 may resist or prevent movement of the top cammed body 326 into the waist portion 446. Accordingly, in some versions, when the guard 404 is moved from the detached position to the attached position (Fig. 16A) the top ledge 445 of the guard 404 may contact the inverted annular cam ledge 338 of the receiving body 312 and prevent or resist additional movement of the guard 404 toward the collection end 308 of the swab stick 302.

In some versions, the second portion diameter 454 may be sized to accommodate the bottom cammed body 322 within the second portion 448 of the guard bore 420. For example, in some versions, the maximum bottom cam diameter 330 may be less than or equal to the second portion diameter 454 adjacent to the waist portion 446. Further, the waist portion diameter 452 may be greater than or equal to the minimum cam diameter 329 but at least slightly less than the maximum bottom cam diameter 330. Accordingly, in some versions, as the guard 404 moves from the detached position to the attached position (from off of the swab stick 302 into the attached position depicted in Fig. 16A), the minimum bottom cam diameter 329 may fit through the waist portion 446 with at least minimal clearance from the waist wall 443. As the guard 404 continues to move toward the collection end 308, the cam wall 323 of the bottom cammed body 322 may contact the waist wall 443 as the diameter of the bottom cammed body 322 increases from the minimum bottom cam diameter 329 to the maximum bottom cam diameter 330. As the diameter of the cammed body 322 increases, the waist wall 443 at the top ledge 445 may begin to interfere with the bottom cam wall 323, resisting further movement of the guard 404 toward the collection end 308. In some versions, when the guard 404 is moved relative to the shaft 307 toward the collection end 308 with enough force to overcome the resistance of the waist wall 443 at the top ledge 445, the bottom cammed body 322 may be pushed past the top ledge 445, through the waist portion 446 and into the bottom portion 448. In other words, in some versions, the maximum bottom cam diameter 330 of the cammed body 322 may be sized so as to have an interference fit with the waist wall 443 of the waist portion 446 that may be overcome when adequate pressure may be applied. In some versions, as the guard 404 may be forced to overcome the interference fit between the waist wall 443 of the waist portion 446, the guard 404 and/or the waist wall 443 may be configured to flex radially outward away from the shaft 307 so as to accommodate passage of the bottom cammed body 322 through the waist portion 446 and into the second portion 448. In such a placement, the inverted annular cam ledge 338 of the top cammed body 126 may rest on the annular top ledge 445 of the guard 404 and the inverted annular bottom ledge 347 may rest on or be proximate the annular bottom cam ledge 336 of the bottom cam portion 322.

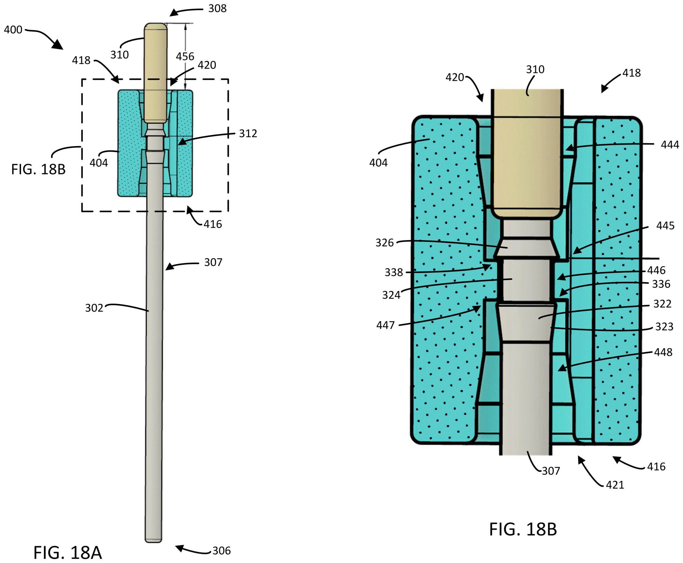

The first portion 444 of the guard bore 420 may have a first length 449 that may be measured longitudinally between the top ledge 445 and the first end 418 of the guard 404. Similarly, the second portion 448 of the guard bore 420 may have a second length 451 measured between the inverted bottom ledge 447 and the second end 416 of the guard 404. In some versions, as is shown more clearly in Figs. 18A and 18B, the first length 449 may determine how much of the collection head 310 may be covered by the guard 404 when installed on the swab stick 302. For example, a longer first length 449 may result in more of the collection head 310 being covered by the guard 404 and less of the collection head 310 being exposed above the first end 318 of the guard 404. Conversely, a shorter first length 449 may result in less of the collection head 310 being covered by the guard 404 and more of the collection head 310 being exposed above the first end 318 of the guard 404. Accordingly, by varying the first length 449, the guard 404 may be used for varying ages and/or sizes of test subjects (e.g., a longer first length 449 for smaller test subjects and shorter top portion length 449 for larger test subjects).

Fig. 18A shows a sectional view of the guard 404 positioned over the receiving body 312 of the swab stick 302 such that the features/geometry of the receiving body 312 may be interlocked with the complimentary interior features formed within the guard bore 420 of the guard 404. Fig. 18B shows a detailed sectional view as indicated in Fig. 18A. In some versions, the guard 404 may be configured to slide over the base end 306 of the swab stick 302, slide up the shaft portion 307, and may lock or snap into place at the receiving body 312. In some versions, an interlocking relationship between the external features of the receiving body 312 and the internal features of the guard 404 may operate to hold the guard 404 in an attached position, such as shown in Fig. 18B.

In some versions, the guard 404 may be configured to be laterally "snapped" onto the swab stick 302 by fitting the swab stick 302 into the guard slot 421. In some versions, the guard 404 may be laterally attached to the swab stick 302 in position over the receiving body 312 as shown in Fig. 16A and 18A. In some versions, the guard 404 may be snapped into position virtually anywhere on the shaft 307 and then be moved toward the collection end 308 until it may be snapped in place over the receiving body 312. In some versions, the guard 404 may slide over the base end 306 of the swab stick 302, slide up the shaft portion 307, and may lock or snap into place at the receiving body 312. In some versions, an interlocking relationship between the external features of the receiving body 312 and the internal features of the guard 404 may operate to hold the guard 404 in an attached position, such as shown in Fig. 18A-B.

In some versions, in the attached position, particular features of the receiving body 312 and the guard 404 may be positioned so as to removably hold the guard 404 in place. For example, in some versions, in the attached position, the guard 404 may be disposed with respect to the shaft 307 such that the recessed body 324 may be disposed within the waist portion 446 of the guard bore 420. In the attached position, the inverted annular cam ledge 338 of the top cammed body 326 may abut or be adjacent to the top ledge 445 of the guard 404 such that the inverted annular cam ledge 338 may prohibit or limit further movement of the guard 404 toward the collection head 310. Conversely, the annular bottom cam ledge 336 of the bottom cammed body 322 may abut or be adjacent to the inverted bottom ledge 447 of the guard 404 such that the annular bottom cam ledge 336 may resist or limit movement of the guard 404 toward the base end 306 of the swab stick 302.

In some versions, the guard 404 may be installed onto the swab stick 302 by inserting the base end 306 of the swab stick into the guard bore 420. The guard 404 may then be moved relative to the shaft 307 from the base end 306 toward the collection end 308. Those skilled in the art will recognize that, alternatively or additionally, moving the swab stick 302 relative to the guard 404 may have the same result as moving the guard 404 relative to the swab stick 302. In some versions, as the guard 404 moves toward the collection end 308, the lower cammed body 322 of the receiving body 312 may pass through the first portion 444 of the guard bore 420 and the top ledge 445 may encounter the cam wall 323 of the bottom cammed body 322. As the diameter of the bottom cammed body 322 increases from the minimum bottom cam diameter 329 to the maximum bottom cam diameter 330, additional movement of the guard 404 may encounter growing resistance. In some versions, when adequate force may be used to move the guard 404 toward the collection end 308, the resistance may be overcome and the bottom cammed body 322 may be received into the second portion 448 of the guard bore 420, as shown in Fig. 18B. In some versions, as the resistance from the bottom cammed body 412 is overcome, the guard 404 may flex radially outward to accommodate passage of the bottom cammed body 422 into the lower portion 448 of the guard bore 420.

In some versions, removing the guard 404 from the attached position to the detached position may be achieved by applying adequate lateral force to the guard 404 laterally away from the swab stick 302 to overcome an interference fit between the recessed body 424 and the waist slot. In some versions, the guard 404 and/or may be configured to flex radially outward away from the shaft 307 so as to accommodate passage of the recessed body 424 through the waist slot 427 to detach from the swab stick 302. In some versions, removing the guard 404 may be alternatively or additionally achieved by applying adequate force to the guard toward the base end 306 of the swab stick 302 to overcome an interference fit between the annular bottom cam ledge 336 of the bottom cammed body 322 and the inverted bottom ledge 447 of the guard 404. In some versions, the guard 404 and/or the waist wall may be configured to flex radially outward away from the shaft 307 so as to accommodate passage of the bottom cammed body 322 through the waist portion 446 and into the second portion 448 (for attachment) or passage into the first portion 444 (for removal).

At least a portion of the collection head 310 may extend clear of the top end 418 of the guard 404, leaving that portion exposed and ready for collecting a specimen from a test subject, which may be defined as an exposed portion of the collection head 310. The exposed portion may be the amount of the collection head 310 that may be inserted into a passage, cavity or other test location (e.g., nasal cavity) of a test subject before being prohibited or resisted from further insertion by the guard 404. Accordingly, the guard 404 may act to protect from inserting the collection head 310 too far into a passage, cavity or other test location (e.g., nasal cavity) of a test cavity. In some versions, the overall length of the guard 404 or at least the first length 449 of the guard may be sized based on how much of the collection head 310 may be left as the exposed portion. For example, in some versions, the exposed portion 456 may be between 50 percent and 90 percent of the collection head 310, or may be between 55 percent and 85 percent in other versions, or between 60 and 80 percent in other versions. In some versions, the exposed portion 456 may be between 30 percent and 70 percent of the collection head 310, or between 40 and 60 percent in other versions.

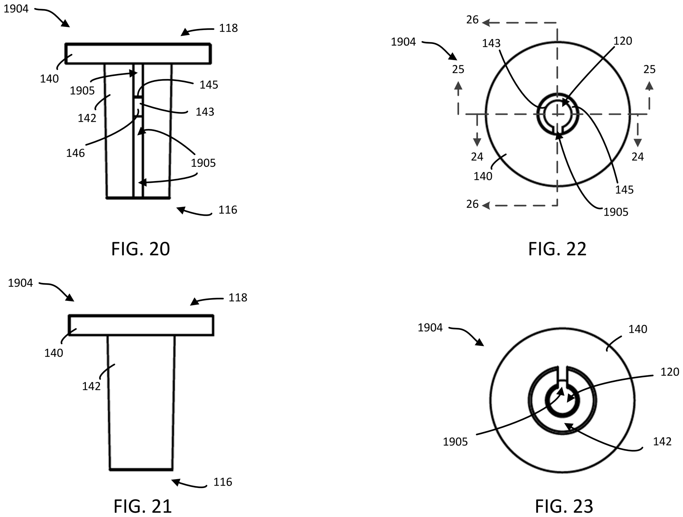

Fig. 19A shows a version of a swab guard system 1900 in a detached position in accordance with the disclosure. Fig. 19B shows the swab guard system 1900 of Fig. 19A in an attached position. Swab guard system 1900 may be identical to swab guard system 100 with the exception that a slit, designated skirt slit 1905, may be disposed within guard 1904, and in particular within skirt 142. With reference to Fig. 20, which shows a front view of the guard 1904; Fig. 21, which shows a rear view, left side view and right side view of the guard 1904; Fig. 22, which shows a top view of the guard 1904; and Fig. 23, which shows a bottom view of the guard 1904, skirt slit 1905 may be disposed in the skirt 142.

For purposes of this portion of description, the skirt 142 may have a height or length that runs longitudinally with respect to swab guard system 1900 (i.e., from the bottom end 116 to the flange 140). The height or length here may generally be understood to be the up and down direction in Fig. 20, for example. The skirt 142 may also have a thickness that may run generally orthogonally with respect to the height or length dimension from an interior-facing surface of the skirt 142, which in part defines the guard bore 120, to an exterior-facing surface of the skirt 142. The height or length of the skirt slit 1905 may be in the same orientation as the height or length of the skirt 142. Likewise, the depth of the skirt slit 1905 may be in the same orientation as the thickness of the skirt 142. The width of the skirt slit 1905 may be the distance between walls that form such skirt slit 1905.

Using these dimensions, the depth of the skirt slit 1905 may extend the entire thickness of the skirt 142 from the exterior-facing surface of skirt 142 to an interior-facing surface, such interior-facing surface defining, at least in part, the guard bore 120 (as depicted). In a version, the depth of the skirt slit 1905 may extend only a portion of the thickness of the skirt 142 (e.g., it may not extend through the entirety of the skirt 142 from an exterior-facing surface of skirt 142 to an interior-facing surface of skirt 142). In such a version, the skirt slit 1905 may be disposed on an exterior-facing surface of the skirt 142 without extending to an interior-facing surface of the skirt 142 or may be disposed on an interior-facing surface of the skirt 142 without extending to an exterior-facing surface of the skirt. In other versions (not depicted), the skirt slit 1905 is made internal to the body of the skirt 142 and does not extend to an interior-facing surface or exterior-facing surface of the skirt 142. In a version, the depth of the skirt slit 1905 may be uniform (as depicted) or may vary across the length of the skirt slit 2705). The skirt slit 1905 may be oriented longitudinally with respect to skirt 142 (as depicted) or may conform to another orientation with respect to skirt 142 (e.g., latitudinal, angled, etc.). In a version, skirt slit 1905 may conform to a straight line (as depicted) or may conform to another shape (e.g., a curved line, a zigzag line, a sinusoidal line, other patterned line, a circle, an teardrop or oval shape, or other geometric form). When oriented longitudinally with respect to skirt 142, the skirt slit 1905 may extend the entire length or height of the skirt 142 from bottom end 116 to flange 140 (as depicted) or may extend only a portion of the length or height of skirt 142. In other versions involving different orientations of the skirt slit 1905, the skirt slit 1905 may extend the entirety of that orientation with respect to skirt 142 (e.g., latitudinally or on another angle) or may extend only a portion of such orientation. The width of the skirt slit 1905 (e.g., the spacing between the respective side walls that form the skirt slit 1905) may be uniform (as depicted) or may vary across the length of the skirt slit 1905.

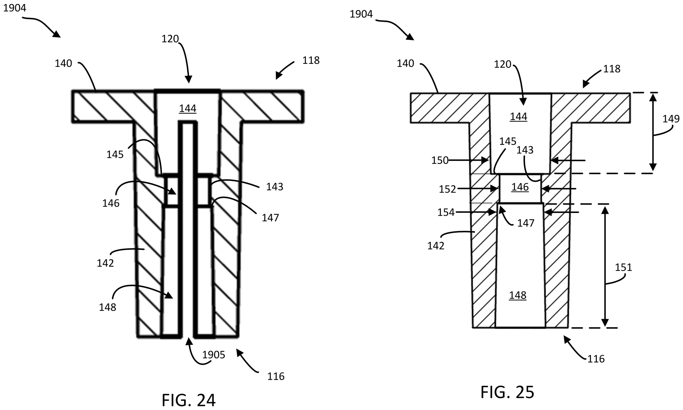

Fig. 24 shows a sectional view of the guard 1904 taken along sectional lines 24:24 as indicated in Fig. 22. Fig. 25 shows a sectional view of the guard 1904 taken along sectional lines 25:25 as indicated in Fig. 22. Fig. 25 is identical to Fig. 4B. Fig. 26 shows a sectional view of the guard 1904 taken along sectional lines 26:26 as indicated in Fig. 22. The face of a side wall associated with skirt slit 1905 is depicted as skirt slit side wall 2602 in Fig. 26. A complementary skirt slit side wall (not depicted) may be adjacently facing skirt slit side wall 2602.

Skirt slit 1905 may be configured to reduce a force necessary to interlock and de-interlock the guard body 1904 and the receiving body 112. For example, skirt slit 1905 may provide increased flexibility to the guard body 1904 to improve the interference fit between the guard body 1904 and the receiving body 112. The skirt slit 1905 may impart a degree flexibility to the guard body 1904 so as to reduce a force necessary to interlock or de-interlock the guard body 1904 and receiving body 112. For example, a force may be applied to the guard body 1904, the receiving body 112, or both the guard body 1904 and the receiving body 112 to cause the guard body 1904 to be interlocked with or de-interlocked from the receiving body 112. The inclusion of skirt slit 1905 in guard body 1904 may increase such flexibility and decrease the force required to cause such interlocking or de-interlocking. Relative to the same guard 1904 and receiving body 112, an increase in a portion of the width (for a non-uniform shaped slit) or in the width of the skirt slit 1905 (for a uniform shaped slit, as depicted) may result in increased flexibility of guard 1904 and reduce the force necessary to interlock or de-interlock the guard body 1904 and the receiving body 112. In a version, the skirt slit 1905 is dimensioned to be less than the width of receiving body 112 or shaft 107. In a version, the width of the skirt slit 1905 is dimensioned to not permit any portion of the swab 102 to be snapped into guard 1904 (or more particularly, into skirt slit 1905) from the side. That is, the skirt slit 1905 may not provide sufficient flexibility for the swab 102 to be snapped into guard 1904 (or more particularly, into skirt slit 1905) from the side.

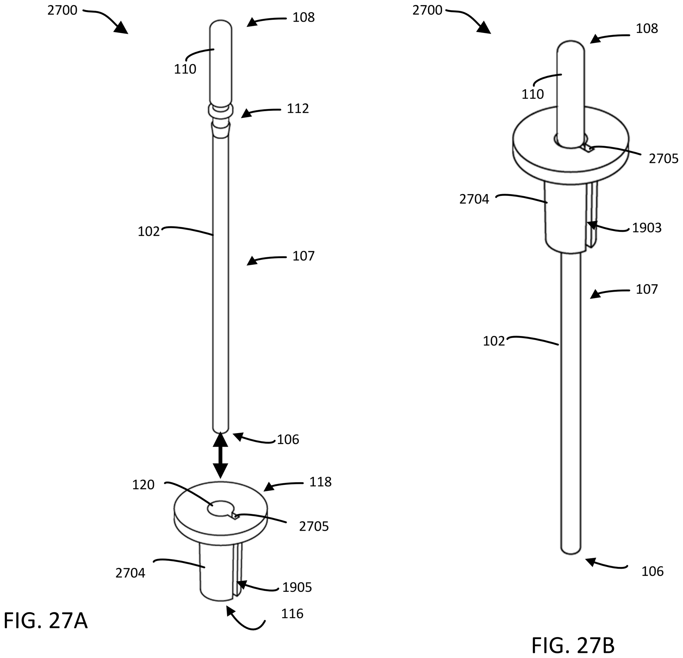

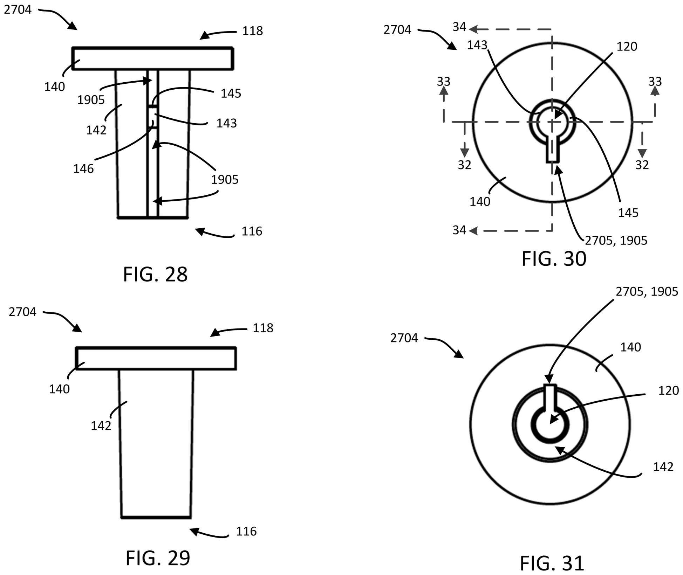

Fig. 27A shows a version of a swab guard system 2700 in a detached position in accordance with the disclosure. Fig. 27B shows the swab guard system 2700 of Fig. 27A in an attached position. Swab guard system 2700 may be identical to swab guard system 1900 with the exception that a slit, designated flange slit 2705, may be disposed within guard 2704. With reference to Fig. 28, which shows a front view of the guard 2704; Fig. 29 which shows a rear view, left side view and right side view of the guard 2704; Fig. 30, which shows a top view of the guard 2704; and Fig. 31, which shows a bottom view of the guard 2704, flange slit 2705 may be disposed within the body of the flange 140 of guard 2704.

For purposes of this portion of description, the height/length and thickness of the skirt 142 may be as described above with respect to Figs. 19A-26, and the flange 140 may have the same dimensions. That is the flange may have a height or length that may run longitudinally with respect to swab guard system 1900 (i.e., from the top end 118 to the skirt 142). The height or length here may generally be understood as the up and down direction in Fig. 28, for example. The flange 140 may also have a thickness that runs generally orthogonally with respect to the height or length dimension from an interior-facing surface of the flange 140, which in part defines the guard bore 120, to an exterior-facing surface of the flange 140. The height or length of the skirt slit 1905 and flange slit 2705 may be in the same orientation as the height or length of the skirt 142 and flange 140, respectively. Likewise, the depth of the skirt slit 1905 and flange slit 2705 may be in the same orientation as the thickness of the skirt 142 and flange 140, respectively. The width of the skirt slit 1905 and flange slit 2705 may be the distance between walls that form each such slit.

Using these dimensions, the depth of the flange slit 2705 may extend the entire thickness of the flange 140 (e.g., from the exterior-facing surface of flange 140 to an interior-facing surface of flange 140, such interior-facing surface defining, at least in part, the guard bore 120). In a version, the depth of the flange slit 2705 may extend only a portion of the thickness of the flange 140 (e.g., it may not extend through the entirety of the flange 140 from an exterior-facing surface of flange 140 to an interior-facing surface of flange 140) (as depicted). In such a version, the flange slit 2705 may be disposed on an exterior-facing surface of the flange 140 without extending to an interior-facing surface of the flange 140 or may be disposed on an interior-facing surface of the flange 140 without extending to an exterior-facing surface of the flange 140 (as depicted). In other versions (not depicted), the flange slit 2705 is made internal to the body of the flange 140 and does not extend to an interior-facing surface or exterior-facing surface of the flange 140. In a version, the depth of the flange slit 2705 may be uniform (as depicted) or may vary across the length of the flange slit 2705). In a version, the flange slit 2705 may have the same depth as the depth of the skirt slit 1905 (as depicted), but it is contemplated that in other versions, flange slit 2705 may have a depth that is different than the depth of the skirt slit 1905. The flange slit 2705 may be oriented longitudinally relative to the length of the guard body 1904 (here, top to bottom) (as depicted) or may conform to another orientation (e.g., latitudinal, angled, etc.). In a version, flange slit 2705 may conform to a straight line (as depicted) or may conform to another shape (e.g., a curved line, a zigzag line, a sinusoidal line, other patterned line, a circle, an teardrop or oval shape, or other geometric form). When oriented longitudinally with respect to the guard body 1904, the flange slit 2705 may extend the entire length or height of the flange 140 from the top end 118 to the skirt 142 (as depicted) or may extend only a portion of the length or height of flange 140. In other versions involving different orientations of the flange slit 2705, the flange slit 2705 may extend the entirety of that orientation (e.g., latitudinally or on another angle) or may extent only a portion of such orientation. The width of the flange slit 2705 (i.e., the spacing between the respective side walls that form the flange slit 2705) may be uniform (as depicted) or may vary across the length of the flange slit 2705. The flange slit 2705 may be aligned (as depicted) or misaligned (e.g., offset) with respect to the position of the skirt slit 1905. If the skirt slit 1905 is aligned with the flange slit 2705 while the skirt slit 1905 is touchingly adjacent to the flange 140 and while the flange slit 2705 is touchingly adjacent to the bottom of the flange 140, then such alignment may form a common slit cavity.

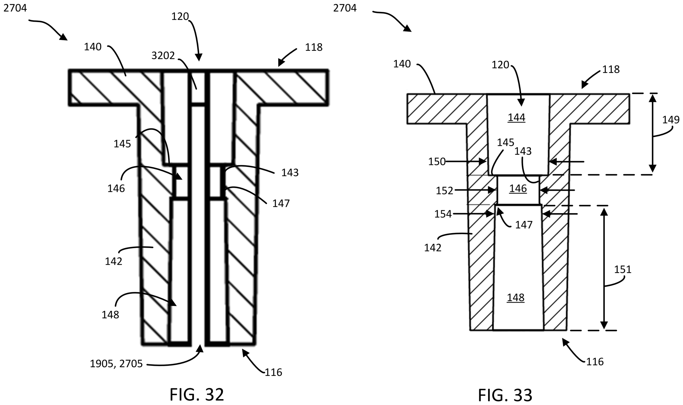

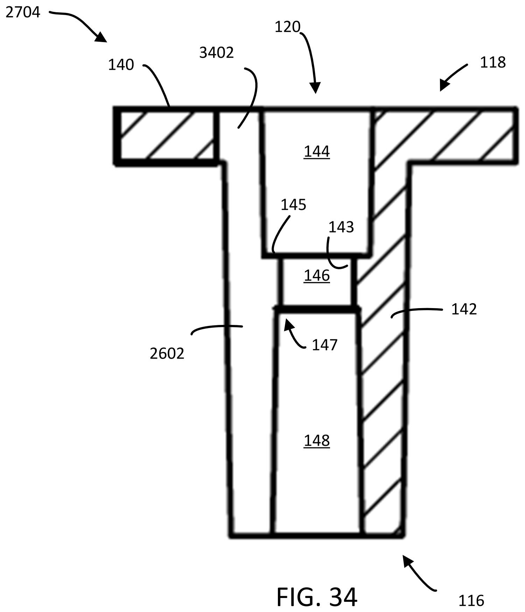

Fig. 32 shows a sectional view of the guard 2704 taken along sectional lines 32:32 as indicated in Fig. 30. Fig. 33 shows a sectional view of the guard 2704 taken along sectional lines 33:33 as indicated in Fig. 30. Fig. 33 is identical to Fig. 4B. Fig. 34 shows a sectional view of the guard 2704 taken along sectional lines 34:34 as indicated in Fig. 30. The face of an edge wall associated with flange slit 2705 is depicted as flange slit edge wall 3202 in Fig. 32. The face of a side wall associated with flange slit 2705 is depicted as flange slit side wall 3402 in Fig. 34. A complementary flange slit side wall (not depicted) may be adjacently facing flange slit side wall 3402. Because the flange slit 2705 is aligned with the skirt slit 1905 in the depicted versions, the flange slit wall 3402 is adjacent to the skirt slit wall 2602. Likewise, because the flange slit 2705 and skirt slit 1905 are aligned in the depicted versions with the slits running the length of the flange 140 and skirt 142, respectively, the top, bottom and sectional views of Figs. 30-32 depict a common slit cavity that extends the length of the guard 2704 from the top end 118 to the bottom end 116. It is recognized that the slits may run only a portion of the length of the flange 140 and skirt 142 and nonetheless form a common slit cavity that runs only a portion of the length of the guard 2704.

Although the version of Figs. 27A-34 depicts both a flange slit 2705 and a skirt slit 1905, it is contemplated that only a flange slit 2705 may be employed in guard 2704 to the exclusion of the skirt slit 1905.

Flange slit 2705 (with or without the skirt slit 1905) may be configured to reduce a force necessary to interlock and de-interlock the guard body 2704 and the receiving body 112. For example, flange slit 2705 (with or without the skirt slit 1905) may provide increased flexibility to the guard body 2704 to improve the interference fit between the guard body 2704 and the receiving body 112. The flange slit 2705 (with or without the skirt slit 1905) may impart a degree flexibility to the guard body 2704 so as to reduce a force necessary to interlock or de-interlock the guard body 1904 and receiving body 112. For example, a force may be applied to the guard body 2704, the receiving body 112, or both the guard body 2704 and the receiving body 112 to cause the guard body 2704 to be interlocked with or de-interlocked from the receiving body 112. The inclusion of flange slit 2705 (with or without the skirt slit 1905) in guard body 2704 may increase such flexibility and decrease the force required to cause such interlocking or de-interlocking. Relative to the same guard 2704 and receiving body 112, an increase in a portion of the width (for a non-uniform shaped slit) or in the width of the flange slit 2705 (for a uniform shaped slit, as depicted) may result in increased flexibility of guard 2704, whether such guard 2704 includes or excludes skirt slit 1905 and reduce the force necessary to interlock or de-interlock the guard body 1904 and the receiving body 112. In a version, the flange slit 2705 is dimensioned to be less than the width of receiving body 112 or shaft 107. In a version, the width of the flange slit 2705 is dimensioned to not permit any portion of the swab 102 to be snapped into guard 2704 (or more particularly, into flange slit 2705) from the side. That is, the flange slit 2705 may not provide sufficient flexibility for the swab 102 to be snapped into guard 2704 (or more particularly, into flange slit 2705) from the side, in each case whether the guard 2704 includes or excludes a skirt slit 1905.

Although not depicted in the Figures, a slit similar to the skirt slit 1905 and flange slit 2705 may be disposed within the versions depicted in Figs. 12-15, and in particular such slit may be disposed within guard 304 as was described above in connection with skirt slit 1905 being disposed within skirt 142 of guard 1904 and guard 2704 and in connection with flange slit 2705 (with or without skirt slit 1905) being disposed within flange 140 of guard 2705. Such a slit may have the same features and characteristics as described above with respect to depth, disposition, uniformity, orientation, shape, length/height, width, and edge and/or side walls. Functionally, such a slit disposed within the body of guard 304 would likewise reduce a force necessary to interlock and de-interlock the guard body 304 and the receiving body 112. For example, such a slit may provide increased flexibility to the guard body 304 to improve the interference fit between the guard body 304 and the receiving body 112. This slit 305 may impart a degree flexibility to the guard body 304 so as to reduce a force necessary to interlock or de-interlock the guard body 304 and receiving body 112.

One of skill in the art will appreciate that the collection end of the swab stick 102 (e.g., collection end 108) may be considered a distal end. Likewise, one of skill in the art will appreciate that the base end of the swab stick 102 (e.g., base end 106) may be considered a proximal end. Throughout this disclosure, particular shapes were described (e.g., annular, circular, cylinder/cylindrical, etc.) Other geometric shapes may be substituted for therefore without deviating from the disclosure. For example, such elements may be semi-annular, semi-circular, semi-cylindrical. In other versions, such elements may be oval-shaped, semi-oval shaped, triangular, semi-triangular, quadrilateral-shaped, semi-quadrilateral-shaped, trapezoidal, semi-trapezoidal, rectangular, semi-rectangular, square, semi-square, pentagonal, semi-pentagonal, hexagonal, semi-hexagonal, octagonal, semi-octagonal, etc., or have cross-sections that are of any of the foregoing geometry. One of skill in the art will further appreciate the various receiving bodies (and the elements described as being part of each receiving body) in the versions of Figs. 1-18 (e.g., receiving body 112) may constitute an integral part of the shaft (e.g., shaft 107), may constitute components disposed on shaft (e.g., 107), or components coupled between shaft (e.g. shaft 107) and collection end (e.g., 108). Similarly, one of skill in the art will appreciate that each of the various geometries described herein (e.g., any collars, any cammed bodies, ledges, etc.) may include axially curved or curved edges to facilitate the placement/removal of guard (e.g., guard 104) at the receiving body (e.g., receiving body 112). Finally, one of skill in the art will appreciate that various aspect, elements, features and geometry of any one version or figure may be used in, used together with, substitute for another aspect, element, feature, or geometry in any other version set forth herein. It is expressly contemplated that such aspects, elements, features and geometries from one version or figure are individually or collectively compatible with or substitute for individual or collective other aspects, elements, features and geometries.

Upon reading this disclosure, those of skill in the art will appreciate still additional alternative structural and functional designs for the systems and methods described herein through the disclosed principles herein. Thus, while particular versions and applications have been illustrated and described, it is to be understood that the disclosed versions are not limited to the precise construction and components disclosed herein. Various modifications, changes and variations, which will be apparent to those skilled in the art, may be made in the arrangement, operation and details of the systems and methods disclosed herein without departing from the spirit and scope defined in any appended claims.

When used in this specification and claims, the terms "comprises" and "comprising" and variations thereof mean that the specified features, steps or integers are included. The terms are not to be interpreted to exclude the presence of other features, steps or components.

Protection may be sought for any features disclosed in any one or more published documents referenced herein in combination with the present disclosure.

Although certain example versions of the invention have been described, the scope of the appended claims is not intended to be limited solely to these embodiments. The claims are to be construed literally, purposively, and/or to encompass equivalents.

The following numbered paragraphs provide further examples of the disclosure:
1. A system comprising:
   a swab stick including a shaft, a collection head, and a receiving body; and
   a guard body including a bore that extends through a longitudinal axis of the guard body,
      wherein:
      the shaft has a distal end and a proximal end,
      the collection head is disposed on the shaft at the distal end of the shaft, the collection head being configured for collecting a sample from a test subject,
      the receiving body is disposed in between the collection head and the proximal end, the receiving body includes a first protrusion relative to the shaft and a second protrusion relative to the shaft, the first and second protrusions defining a recess therebetween, at least one of the first protrusion and the second protrusion is a cammed body,
      the bore is sized and shaped to accept the shaft, the bore includes a first portion, a second portion, and a third portion, the first portion having a shape that geometrically complements the first protrusion, the second portion having a shape that geometrically complements the recess and the third portion having a shape that geometrically complements the second protrusion, and
      the geometrically complementing portions of the receiving body and the corresponding first protrusion, recess and second
      protrusion of the receiving body permit the guard body to be removably interlocked with the receiving body.
2. The system of clause 1, wherein:
   the test subject is one of an adult, a child, and an infant, and
   the collection head shaped and sized to be inserted into a nasal passage of the test subject and the collection head is configured for collecting a sample from said nasal passage.
3. The system of any preceding clause, wherein the collection head comprises one of a polyurethane sponge, cotton, poly fibers, and flocked fibers.
4. The system of any preceding clause, wherein:
   the guard body includes a top end and a bottom end, and
   the guard body includes a flange disposed at the top end and a skirt disposed at the bottom end.
5. The system of clause 4, wherein the guard body includes a slot oriented parallel to the longitudinal axis of the guard body, the slot being sized and shaped to permit the receiving body to be inserted into the bore through the slot.
6. The system of any of clauses 1-3, wherein:
   the guard body includes a top end and a bottom end, and
   the guard body is shaped as a cylinder.
7. The system of clause 6, wherein the guard body includes a slot oriented parallel to the longitudinal axis of the guard body, the slot being sized and shaped to permit the receiving body to be inserted into the bore through the slot.
8. The system of any preceding clause, wherein the shaft is one of axial and non-axial.
9. The system of any preceding clause, wherein the recess has the same diameter as the shaft.
10. The system of any preceding clause, wherein the receiving body is formed integrally with the shaft.
11. The system of any preceding clause, wherein the first protrusion is a cammed body and the second protrusion is a proud collar.
12. The system of any preceding clause, wherein the first protrusion is a cammed body and the second protrusion is a cammed body.
13. The system of clause 12, wherein the first protrusion is inverted with respect to the second protrusion.
14. The system of clause 13, wherein the first protrusion has a cammed wall that tapers toward the proximate end of the shaft and the second protrusion has a cammed wall that tapers toward the distal end of the shaft.
15. The system of any preceding clause, wherein:
   the first portion is a top portion, the second portion is a waist portion and the third portion is a bottom portion,
   the first portion and the waist portion define an annular top ledge, and
   the waist portion and the second portion define an inverted annular bottom ledge.
16. The system of clause 15, wherein:
   the top portion has a diameter, the waist portion has a diameter, and the bottom portion has a diameter,
   the difference between the top portion diameter and the waist portion diameter forms the annular top ledge, and
   the difference between the waist portion diameter and the bottom portion diameter forms the inverted annular bottom ledge.
17. The system of clause 15, wherein:
   the first protrusion is a cammed body having an annular cam ledge,
   the second protrusion is a proud collar having an annular collar ledge,
   when the guard body is interlocked with the receiving body, the proud collar is disposed in the top portion and the annular collar ledge abuts the annular top ledge and the cammed body is disposed within the bottom portion with the annular cam ledge proximate the inverted annual bottom ledge.
18. The system of clause 17, wherein the waist portion has a waist wall and when the guard body is interlocked with the receiving body, the waist wall is interlocked with the recess.
19. The system of clause 4, wherein:
   the first portion is a top portion, the second portion is a waist portion, and the third portion is a bottom portion,
   the bottom portion accommodates the cammed body, and
   one of:
      the bottom portion does not accommodate the other protrusion, and
      the bottom portion only partially accommodates the other protrusion.
20. The system of clause 4, wherein:
   the guard body has a length such that when the collection head is inserted into
   the guard body, the length of the guard body covers one of:
   the entirety of the collection head, and
   at least a portion of the collection head.
21. A system comprising:
   a swab stick including a shaft, a collection head, and a receiving body; and
   a guard body including a bore that extends through a longitudinal axis of the guard body and a slit,
      wherein:
      the shaft has a distal end and a proximal end,
      the collection head is disposed on the shaft at the distal end of the shaft, the collection head being configured for collecting a sample from a test subject,
      the receiving body is disposed in between the collection head and the proximal end,
      the bore is configured to accept the shaft, the bore having a shape that geometrically complements a shape of the receiving body to permit the guard body to be removably interlocked with the receiving body, and
      the slit is configured to reduce a force necessary to interlock and de-interlock the guard body and receiving body.
22. The system of clause 21, wherein:
   the test subject is one of an adult, a child, and an infant, and
   the collection head is shaped and sized to be inserted into a nasal passage of the test subject and the collection head is configured for collecting a sample from said nasal passage.
23. The system of clause 21 or 22, wherein:
   the guard body includes a top end and a bottom end, and
   the guard body includes a flange disposed at the top end and a skirt disposed at the bottom end.
24. The system of clause 23, wherein the slit is disposed in the skirt.
25. The system of clause 23 or 24, wherein the slit is oriented longitudinally with respect to the skirt.
26. The system of any of clauses 23-25, wherein the slit extends the length of the skirt.
27. The system of any of clauses 23-25, wherein the depth of the slit extends from an exterior-facing surface of the skirt to an interior-facing surface of the skirt.
28. The system of clause 23, wherein the slit is disposed in the flange.
29. The system of clause 28, wherein the slit is disposed on an interior-facing surface of the flange.
30. The system of clause 23, wherein the slit comprises a skirt slit disposed in the skirt and a flange slit disposed in the flange.
31. The system of clause 30, wherein the skirt slit is oriented longitudinally with respect to the skirt.
32. The system of clause 30 or 31, wherein the skirt slit extends the length of the skirt.
33. The system of any of clauses 30-32, wherein the depth of the skirt slit extends from an exterior-facing surface of the skirt to an interior-facing surface of the skirt.
34. The system of any of clauses 30-33, wherein the flange slit is disposed on an interior-facing surface of the flange.
35. The system of any of clauses 30-34, wherein the skirt slit and the flange slit are aligned and form a common slit cavity.
36. The system of any of clauses 21-35, wherein the width of the slit is less than the width of the shaft.
37. The system of clause 21 or 22, wherein:
   the guard body includes a top end and a bottom end, and
   the guard body is shaped as a cylinder.
38. The system of clause 37, wherein the slit extends the length of the guard body.
39. The system of clause 37 or 38, wherein the slit is disposed on an interior-facing surface of the guard body.
40. The system of any of clauses 37-39, wherein the width of the slit is less than the width of the shaft.
1A. A system comprising:
   a swab stick including a shaft, a collection head, and a receiving body; and
   a guard body including a bore that extends through a longitudinal axis of the guard body and a slit,
      wherein:
      the shaft has a distal end and a proximal end,
      the collection head is disposed on the shaft at the distal end of the shaft, the collection head being configured for collecting a sample from a test subject,
      the receiving body is disposed in between the collection head and the proximal end,
      the bore is configured to accept the shaft, the bore having a shape that geometrically complements a shape of the receiving body to permit the guard body to be removably interlocked with the receiving body, and
      the slit is configured to reduce a force necessary to interlock and de-interlock the guard body and receiving body.
2A. The system of clause 1A, wherein:
   the test subject is one of an adult, a child, and an infant, and
   the collection head is shaped and sized to be inserted into a nasal passage of the test subject and the collection head is configured for collecting a sample from said nasal passage.
3A. The system of clause 1A, wherein:
   the guard body includes a top end and a bottom end, and
   the guard body includes a flange disposed at the top end and a skirt disposed at the bottom end.
4A. The system of clause 3A, wherein the slit is disposed in the skirt.
5A. The system of clause 4A, wherein the slit is oriented longitudinally with respect to the skirt.
6A. The system of clause 5A, wherein the slit extends the length of the skirt.
7A. The system of clause 5A, wherein the depth of the slit extends from an exterior-facing surface of the skirt to an interior-facing surface of the skirt.
8A. The system of clause 3A, wherein the slit is disposed in the flange.
9A. The system of clause 8A, wherein the slit is disposed on an interior-facing surface of the flange.
10A. The system of clause 3A, wherein the slit comprises a skirt slit disposed in the skirt and a flange slit disposed in the flange.
11A. The system of clause 10A, wherein the skirt slit is oriented longitudinally with respect to the skirt.
12A. The system of clause 10A, wherein the skirt slit extends the length of the skirt.
13A. The system of clause 10A, wherein the depth of the skirt slit extends from an exterior-facing surface of the skirt to an interior-facing surface of the skirt.
14A. The system of clause 10A, wherein the flange slit is disposed on an interior-facing surface of the flange.
15A. The system of clause 10A, wherein the skirt slit and the flange slit are aligned and form a common slit cavity.
16A. The system of clause 1A, wherein the width of the slit is less than the width of the shaft.
17A. The system of clause 1A, wherein:
   the guard body includes a top end and a bottom end, and
   the guard body is shaped as a cylinder.
18A. The system of clause 17A, wherein the slit extends the length of the guard body.
19A. The system of clause 17A, wherein the slit is disposed on an interior-facing surface of the guard body.
20A. The system of clause 17A, wherein the width of the slit is less than the width of the shaft.

## Claims

1. A system comprising:
a swab stick including a shaft, a collection head, and a receiving body; and
a guard body including a bore that extends through a longitudinal axis of the guard body,
wherein:
the shaft has a distal end and a proximal end,
the collection head is disposed on the shaft at the distal end of the shaft, the collection head being configured for collecting a sample from a test subject,
the receiving body is disposed in between the collection head and the proximal end,
the bore is configured to accept the shaft, the bore having a shape that geometrically complements a shape of the receiving body to permit the guard body to be removably interlocked with the receiving body.

2. The system of claim 1, wherein the guard body further includes a slit, wherein the slit is configured to reduce a force necessary to interlock and de-interlock the guard body and receiving body.

3. The system of claim 2, wherein:
the test subject is one of an adult, a child, and an infant, and
the collection head is shaped and sized to be inserted into a nasal passage of the test subject and the collection head is configured for collecting a sample from said nasal passage.

4. The system of claim 2 or 3, wherein:
the guard body includes a top end and a bottom end, and
the guard body includes a flange disposed at the top end and a skirt disposed at the bottom end.

5. The system of claim 4, wherein the slit is disposed in the skirt.

6. The system of claim 4 or 5, wherein the slit is oriented longitudinally with respect to the skirt.

7. The system of any of claims 4-6, wherein the slit extends the length of the skirt.

8. The system of any of claims 4-7, wherein the depth of the slit extends from an exterior-facing surface of the skirt to an interior-facing surface of the skirt.

9. The system of claim 4, wherein the slit is disposed in the flange.

10. The system of claim 9, wherein the slit is disposed on an interior-facing surface of the flange.

11. The system of claim 4, wherein the slit comprises a skirt slit disposed in the skirt and a flange slit disposed in the flange.

12. The system of claim 11, wherein:
the skirt slit is oriented longitudinally with respect to the skirt and/or
the skirt slit extends the length of the skirt; and/or
the depth of the skirt slit extends from an exterior-facing surface of the skirt to an interior-facing surface of the skirt; and/or
the flange slit is disposed on an interior-facing surface of the flange; and/or
the skirt slit and the flange slit are aligned and form a common slit cavity.

13. The system of any of claims 2-12, wherein the width of the slit is less than the width of the shaft.

14. The system of claim 2 or 3, wherein:
(i) the guard body includes a top end and a bottom end, and the guard body is shaped as a cylinder; and/or
(ii) the slit extends the length of the guard body; and/or
(iv) the slit is disposed on an interior-facing surface of the guard body; and/or
(v) the width of the slit is less than the width of the shaft.
